# EUROPEAN PATENT APPLICATION

(11) **EP 3 653 215 A1**
(43) Date of publication of application: **20.05.2020**
(21) Application number: 19386046.7
(22) Date of filing: 14.11.2019
(51) Int. Cl.: A61K 31/66, A61K 49/00, A61P 17/00, A61P 35/00, C07F 9/40

(54) **THERANOSTIC INHIBITORS AND ACTIVITY-BASED PROBES FOR THE KLK7 PROTEASE**

(30) Priority: 16.11.2018 GR 20180100521
(71) Applicant: University of Patras, 26504 Rio Patras (GR); Sotiropoulou, Georgia, 26500 Rio Patras (GR); Pampalakis, Georgios, 26500 Ovrya Patras (GR); Bisyris, Evaggelos, 26441 Patras (GR)
(72) Inventor: Sotiropoulou, Georgia, 26500 Rio Patras (GR); Pampalakis, Georgios, 26500 Ovrya Patras (GR); Bisyris, Evaggelos, 26441 Patras (GR)

(57) **Abstract**

The present invention provides specific, selective and irreversible inhibitors and activity-based probes (ABPs) of KLK7. These ABPs and inhibitors are useful as new treatments for skin desquamating and/or inflammatory disorders, as new antiinflammatory agents, new anti-neurodegenerative agents and as new anticancer therapeutics. Also provided are pharmaceutical combinations of the compounds according to the invention with inhibitors of KLK5 for use in the treatment of said diseases and disorders. The invention also provides cosmetic compositions for skin application, preferably for the improvement of an undesirable skin condition. The compounds of the present invention can also be used for the *in vitro* and *in vivo* molecular diagnosis of skin disorders characterized by abnormal desquamation and/or inflammation, inflammatory diseases, neurodegenerative diseases or cancer.

## Description

### TECHNICAL FIELD

The invention relates to compounds that are highly specific and selective inhibitors and activity-based probes for the KLK7 serine protease. These compounds are useful for the treatment of diseases such as skin disorders characterized by abnormal desquamation and/or inflammation, other inflammatory diseases, neurodegenerative diseases and cancer, and are also useful in the detection of the active KLK7 protease *in vitro* and *in vivo.*

### BACKGROUND OF THE INVENTION

Kallikrein-related peptidases (KLKs) are the largest serine protease family in the human genome encompassing fifteen highly conserved members. All KLKs are trypsin or chymotrypsin-like enzymes. KLKs participate in various physiological and pathological conditions such as skin desquamation, semen liquefaction, cancer (displaying either tumor promoting or tumor suppressing action), neurodegeneration and others. Furthermore, KLKs participate in inflammatory processes and in the function of the immune system. Given their important roles, KLKs have been investigated as drugs (*e.g*. pharmaceutical proteins or virally-mediated KLK expression) or drug targets (for inhibition).

Kallikrein-related peptidase 7 (KLK7: human; Klk7: mouse; Uniprot: P49862 human; Q91VE3 mouse) is a chymotrypsin-like serine protease originally identified in the skin, where it is transported and secreted with the lamellar granule system at the superficial stratum granulosum. It is recognized that KLK7 is a member of a proteolytic cascade that regulates the epidermal desquamation process. In this cascade, active KLK5 removes the pro-peptide from the KLK7 zymogen, resulting in KLK7 activation. In the epidermis, the activity of KLK7 is mainly regulated by the endogenous inhibitor LEKTI (Lympho-Epithelial Kazal-Type-related Inhibitor) in a pH-dependent manner. In the epidermis, KLK7 cleaves the proteins corneodesmosin and desmocollin 1 that are components of corneodesmosomes and degrades the lipid processing enzymes β-glucocerebrosidase and acidic sphingomyelinase. Also, KLK7 cleaves pro-IL-1β and releases the active cytokine IL-1β, and can also cleave and activate/deactivate cathelicidins.

KLK7 activity has been linked to various inflammatory and/or desquamating skin disorders. KLK7 has been found upregulated in lesions from psoriatic patients. Epidermal overexpression of KLK7 in mice (Tg-KLK7) results in epidermal inflammation, scales, epidermal hyperplasia, hyperkeratosis, and itchy phenotype. Extensive similarities between Tg-KLK7 mice and chronic lesions of atopic dermatitis in humans are well-established and increased KLK7 has been found in atopic dermatitis lesions. A 3'-UTR polymorphism in the KLK7 gene has been identified in some atopic dermatitis patients which is associated with increased *KLK7* mRNA expression. However, other studies have failed to provide an association of this polymorphism with atopic dermatitis. KLK7 upregulation has been identified upon filaggrin knockdown in skin equivalent models (atopic dermatitis model). TH2-type cytokines are also responsible for the increased KLK7 expression in atopic dermatitis. Another study has suggested that in atopic dermatitis there is impaired KLK7 secretion from lamellar granules.

Netherton syndrome (NS) patients show increased KLK5 and KLK7 expression in the skin. Deletion of *Klk5* gene on the *Spink5*-null background (a mouse model of NS) results in rescue of their lethal phenotype in the neonatal phase (Furio et al. PLoS Genet 11: e1005389, 2015). *Klk5*^{*-*/}*⁻Spink5*^{*-*/*-*} mice appear normal at birth and up to P3, when they develop severe scaling and inflammation and succumb on P7. In triple knockout *Klk7*^{*-*/}*⁻Klk5*^{*-*/}*⁻Spink5*^{*-*/*-*} mice, however, the NS-like phenotype is completely rescued (Kasparek et al. PLoS Genet 13: e1006566, 2017).

Altered KLK7 expression has been found in various cancers. KLK7 is overexpressed in papillary thyroid, colon and ovarian cancer. Serum KLK7 is increased in serous and papillary serous ovarian cancer patients and is associated with paclitaxel resistance. KLK7 is a part of the OVSCORE algorithm that predicts surgical outcome in ovarian cancer. KLK7 is highly expressed in pancreatic cancer where it enhances cell invasion by cleaving E-cadherin. Lentiviral mediated knock down of KLK7 in pancreatic cells reduced their proliferation, migration and invasion. KLK7 predicts poor prognosis in patients with non-resectable pancreatic ductal adenocarcinoma. Aberrant KLK7 expression in melanoma cells switches their phenotype from proliferative to invasive. Increased serum KLK7 levels have been found in cervical cancer patients, while the expression of KLK7 is increased with advanced severity of cervical neoplasias. KLK7 displays increased immunoreactivity in gliomas and is associated with poor patient prognosis. KLK7 is downregulated in the serum of breast cancer patients and in prostate cancer cells compared to normal and benign counterparts. However, ectopic expression of KLK7 in prostate cancer cells promotes invasion and metastasis. KLK7 immunohistochemical staining shows strong expression in low grade clear cell renal cell carcinoma (RCC) compared to high grade and it can distinguish between oncocytoma and chromophobe RCC. Early stage oral squamous cell carcinoma is associated with the rs10581213 polymorphism in the KLK7 gene.

KLK7 has been associated with Alzheimer's disease. Specifically, it was demonstrated that KLK7 is an Aβ degrading enzyme, KLK7 expression in decreased in the brains of Alzheimer's disease patients and *Klk7*^{*-*/*-*} mice display 1.4-2-fold increased Aβ. Further, deletion of Klk7 gene in a mouse model of Alzheimer's disease exacerbated amyloid deposition. Cleavage of Aβ peptides *in vitro* by KLK7 inhibits their fibrilization and reduces their neuronal toxicity. Also, decreased concentration of KLK7 is found in cerebrospinal fluid of frontotemporal dementia and Alzheimer's patients.

Very recently, Klk7 expression levels have been found increased in the brains of mouse models of Parkinson's disease (PD). Silencing of Klk7 resulted in increased concentrations of neurotransmitters, decreased expression of proinflammatory cytokines, and decreased brain cell apoptosis. Further silencing of Klk7 improves PD symptoms in PD mice. In addition, it increases SOD1 levels in the brains of PD mice, suggesting that Klk7 could be involved in the SOD1 cycle, so it may functionally correlate with amyotrophic lateral sclerosis (Zhang et al., 2019 J Mol Neurosci 69: 197-214).

KLK7 cleaves human insulin A and B-chains. It appears to regulate body weight *in vivo* since the *Klk7*^{*-*/*-*} mice exhibit lower body fat and display significantly lower serum leptin levels. Deletion of Klk7 gene attenuates adipose tissue inflammation in high fat diet, increases energy expenditure, and improves fatty acid oxidation.

Given the important roles of KLK7 in various disorders it is necessary to have a reliable assay to determine the concentration of the active enzyme and to develop new specific and selective KLK7 inhibitors for pharmacological intervention. Previous attempts to synthesize or identify natural small molecule KLK7 inhibitors have been carried out, however, they displayed: (a) lack of specificity, (b) reversible mode of binding, and/or (c) insufficient potency. For instance, US Patent 20130172267 claims that cyclic depsipeptides isolated from *Chondromyces crocatus* bacterium can inhibit KLK7 activity but they are not selective. Other natural KLK7 inhibitors are the isocoumarin derivatives that can also inhibit KLK5. 1-acyl-1,2,4-triazole derivatives are synthetic covalent but reversible inhibitors that are not selective for KLK7. Although these inhibitors bind covalently to the catalytic Ser, the acylation is reversible and can be hydrolyzed spontaneously after 6 hours. 3-carboxylate coumarin derivatives are KLK5, KLK7, KLK14 and matriptase inhibitors. KLK5, KLK14 and matriptase are inhibited reversibly while KLK7 is inhibited irreversibly. These inhibitors can reduce the epidermal overproteolysis in skin biopsy sections from transgenic KLK5 mice. A quinazolinone derivative has been identified by virtual screening as a reversible inhibitor of KLK7. Although this appeared to be specific for KLK7 compared to other proteases tested (KLK5, KLK14, matriptase) it has not been tested *in vivo* or in *in vitro* models. Other reversible inhibitors of KLK7 include pyridoimidazodiazepinone derivatives, 1,3,6-trisubstituted 1,4-diazepan-7-ones, and isomannide derivatives. Certain brintonamides from a marine cyanobacterium can inhibit KLK7 but also all other proteases (chymase, caspase 14 and chymotrypsin) tested.

Thus it becomes apparent that there is a need for selective, irreversible and potent inhibitors of KLK7 that can discriminate between KLK7 and the other family members, for the treatment of diseases and syndromes with a KLK7 involvement, as well as for the reliable detection and quantification of the active enzyme. The present invention addresses all these needs.

### SUMMARY OF THE INVENTION

The invention relates to specific and selective inhibitors of kallikrein-related peptidase 7 (KLK7) activity tested *in vitro* and *in vivo.* The developed KLK7 inhibitors can be used as pharmaceutical compounds for the development of drugs for treatment of diseases in which KLK7 activities are implicated, as for example rare and common skin diseases characterized by inflammation and/or abnormal desquamation, inflammatory diseases, neurodegenerative diseases and certain types of cancer (colorectal, prostate, ovarian, and others).

Thus, in a first embodiment, the present invention provides a compound of formula (I) 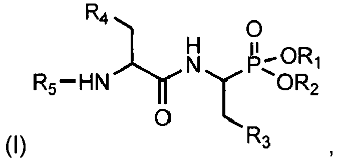 wherein R1, R2, R3, R4 and R5 are as specifically defined herein, or pharmaceutically acceptable salt, isomer, enantiomer, tautomer or racemic mixture thereof, for use in the treatment of a skin disorder characterized by abnormal desquamation and/or inflammation, an inflammatory disease, a neurodegenerative disease or cancer.

In certain embodiments, the compounds claimed in the present invention carry a detectable label or can be modified using click chemistry to incorporate a detectable label. These compounds are useful as activity-based probes (ABPs) specific for the active form of KLK7. Therefore, the invention also provides methods for analysis, molecular diagnosis and *in vivo* imaging using these KLK7-ABPs. These novel KLK7-ABP inhibitors can be exploited for the detection and quantification of active forms of KLK7 protease in biological and clinical specimens for diagnostic and research applications, for the in vivo imaging of active forms of KLK7, as well as for the in vivo detection of the compounds of the present invention after administration to the subject (theranostic applications).

The present invention also provides cosmetic compositions for application to the skin, preferably for the improvement of undesirable skin conditions.

Other aspects and further scope of applicability of the present invention will become apparent from the detailed description to follow.

### BRIEF DESCRIPTION OF THE DRAWINGS

The present invention will now be described with reference to certain embodiments thereof which are illustrated in the accompanying drawings. It should be noted that the accompanying drawings illustrate preferred embodiments of the invention, therefore should not be considered as limiting the scope of the invention.
Fig. 1 shows the results from a gel zymography (casein) assay demonstrating that Boc-FFP can only inhibit the activity of KLK7.
Fig. 2 is a Western-blot assay for (A) the detection of active KLK7 with the ABP Biotin-FFP, whereas (B) shows that Biotin-FFP does not react with KLK6 and trypsin.
Fig. 3 shows the results from a gel zymography (casein) assay demonstrating that only Boc-FFP can inhibit the activity of KLK7 compared to Boc-FbFP and Boc-FcFP.
Fig. 4 shows the results from a western-like assay showing Biotin-FFP labeling of KLK7 versus labeling of KLK7 with Biotin-FbFP. The Biotin-FFP labeling results in a significant signal enhancement.
Fig. 5 are photographs of skin cryosections of normal skin and skin from Netherton syndrome patients, subjected to activography with the KLK7-specific ABP showing tissue localization of KLK7.
Fig. 6 shows photographs of cryosections from a TPA-treated mouse epidermis where active KLK7 is detected using activography
Fig 7 is (A) a schematic diagram of the ELISA assay, and (B) representative analysis of active KLK7 using the ELISA assay.
Fig. 8 shows (A) macroscopic images of *Spink5*^{*-*/}*⁻Klk5*^{*-*/*-*} mice treated with the KLK7 inhibitor. The area included inside the line is the area that received the treatment. Solvent control was added on the left side of the mice and the inhibitor on the right side; and (B) quantification of the observed significant reduction of desquamation.
Fig. 9 shows the expression of inflammatory cytokines in *Spink5*^{*-*/}*⁻Xlk5*^{*-*/*-*} mice, determined by RT-qPCR. The black bar shows solvent treatment, the white bar shows inhibitor treatment.
Fig. 10 shows that Boc-FFP and free (non-protected) amino-FFP (H₂N-FFP) inhibit the KLK7 activity as determined by gel zymography.

### DETAILED DESCRIPTION OF THE INVENTION

As used herein, an "activity-based probe" is a chemical probe that directly detects the formation of a catalytically active site in an enzyme.

As used herein, the term "click chemistry" refers to a method for attaching a probe or substrate (such as an enzyme substrate) of interest to a specific bio-macromolecule or small molecule. Similarly, as used throughout the description and claims, a "click-reactive functional group" refers to a functional group that has click reactivity and/or can undergo a click reaction with a complementary click reactive functional group or molecule. Examples of the types of reactions that are known to have click reactivity include cycloaddition reactions, nucleophilic substitution, radical additions, Michael additions, as well as Diels-Alder and retro Diels-Alder reactions to name a few.

The term "branched or unbranched C₁₋₆ alkyl" as used therein refers to a hydrocarbon chain of carbon atoms linked together by single bonds, wherein the chain may be unbranched (straight chain) or branched and does not include cyclic residues.

Examples of C₁₋₆ alkyl groups are, without limitation, methyl, ethyl, propyl, isopropyl and 2-methylpropyl groups.

The term "C₁₋₆ heteroalkyl" as used therein refers to a "C₁₋₆ alkyl" as defined herein wherein one or more atoms of the "C₁₋₆ alkyl" moiety are substituted by one or more heteroatoms selected from N, O, S and P.

The term "C₂₋₆ alkenyl" as used therein refers to a branched or unbranched hydrocarbon group of 2 to 6 carbon atoms containing at least one carbon-carbon double bond, such as ethenyl, n-propenyl, isopropenyl, n-butenyl, isobutenyl and the like.

The term "C₂₋₆ alkynyl" as used herein refers to a branched or unbranched hydrocarbon group of 2 to 6 carbon atoms containing at least one -C≡C- bond, such as ethynyl, *n-*propynyl, isopropynyl, *n*-butynyl and the like.

The term "C₅₋₁₁ cycloalkyl" as used herein refers to an aliphatic (non-aromatic) ring system having 5 to 11 carbon atoms and 1 to 3 rings, including cyclopentyl, cyclohexyl and adamantyl.

The term "heterocyclyl" as used herein refers to a nonaromatic 3- to 11- membered ring containing at least one endocyclic N, O, or S atom, whereas heterocyclyloxy refers to a heterocyclyl group, as defined herein, attached to the parent molecule through an oxygen atom.

The term "C₆₋₁₄ aryl" as used herein refers to a single aromatic ring such as a phenyl ring, or two or more aromatic rings that are fused together, for instance a naphthyl or anthracenyl group, whereas the term "heteroaryl" includes, without limitation, a thienyl, furanyl, pyrrolyl, imidazolyl, pyrazolyl, triazolyl, tetrazolyl, oxazolyl, isoxazolyl, oxadiazolyl, thiazolyl, isothiazolyl, thiadiazolyl, pyridyl, pyridazinyl, pyrimidinyl, pyrazinyl, indolizinyl, purinyl, naphthyridinyl, and pteridinyl group.

C₁₋₆ alkoxy refers to a C₁₋₆ alkyl-O-group, wherein the alkyl group is as defined herein. Examples of alkoxy groups are, without limiting the range of options, methoxy, ethoxy, methoxyethyl, propoxy, 2-propoxy, butoxy, tertiary butoxy, pentyloxy and hexyloxy group.

C₁₋₆ acyl refers to an alkyl group attached to the parent molecule through a -(C=O)-linkage and C₂₋₆ acyloxy, refers to an alkyl group attached to the parent molecule through an oxygen linkage.

C₁₋₆ alkoxycarbonyl refers to a C₁₋₆ alkoxy group attached to the parent molecule through a carbonyl group. Representative examples of C₁₋₆ alkoxycarbonyl include, but are not limited to, methoxycarbonyl, ethoxycarbonyl, and tert-butoxycarbonyl.

The term "fluorescent quencher" refers to a moiety capable of absorbing the fluorescence energy of an excited reporter molecule, thereby quenching the fluorescence signal that would otherwise be released from the excited reporter molecule.

The term "amide bond" refers to the chemical bond formed between the amino and carboxyl functional groups of two moieties. Boc (tert-Butyloxycarbonyl protecting group), Fmoc (fluorenylmethyloxycarbonyl protecting group) and Z-group (Benzyloxycarbonyl group) refer to amine protecting groups used in synthetic chemistry.

The term "responsiveness" when used in reference to a treatment refers to the degree of effectiveness of the treatment in lessening or decreasing the symptoms of a disease. Furthermore, the term "responsiveness" also refers to the sensitivity of an individual cell, a cell population, a cluster of cells, a tissue, an organ or an organism to the effects of the treatment.

The inventors have surprisingly found that certain phosphonates specifically and selectively react with and bind irreversibly to the catalytic residues of active KLK7 enzyme. Thus, the compounds of the present invention can selectively inhibit the active form of KLK7 without interfering with the function of the other family members. Said compounds are useful in the treatment or prevention of diseases or disorders where the active KLK7 protease is implicated in the pathology of the disease or disorder. As will also become obvious from the Examples herein, the compounds according to the invention are particularly useful in the treatment or prevention of skin disorders characterized by abnormal desquamation and/or inflammation, inflammatory diseases and cancer.

In one aspect, the present invention provides compounds of Formula (I): 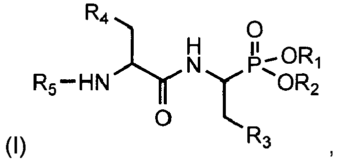 wherein
R1 and R2 are independently selected from branched or unbranched: C₁₋₆alkyl; heteroalkyl; C₂₋₇ alkenyl; C₂₋₇ alkynyl; C₆₋₁₁ cycloalkyl; heterocyclyl; C₆₋₁₁ aryl; C₆₋₁₁ aryl-C₁₋₆ alkyl; heteroaryl; heteroaryl-C₁₋₆ alkyl; C₁₋₆ alkoxy; heterocyclyloxy; C₂₋₇ acyl; C₂₋₇ acyloxy; C₁₋₆ alkoxycarbonyl; naphthyl; or anthracenyl group; wherein each may be unsubstituted or substituted by one or more of halogen, hydroxy, amino, aminoalkyl, thiol, cyano, nitro, oxo, carboxy, C₁₋₃ alkyl, carboxyalkyl or C₁₋₃ alkoxy group; or R1 is C₁₋₂₀ alkyl or C₃₋₂₀ cycloalkyl group, preferably methyl, ethyl, propyl or isopropyl and R2 is a C₆₋₁₁ aryl that may be unsubstituted or substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher;
R3 and R4 are independently selected from phenyl, para-hydroxyphenyl, para-fluorophenyl, para-trifluoromethyl, pentafluorophenyl, 2,4-difluorophenyl, 2,3-difluorophenyl, 3,4-difluorophenyl, 2,5-difluorophenyl, 2,6-difluorophenyl, 2,3,4-trifluorophenyl, 2,3,5-trifluorophenyl, 2,3,6-trifluorophenyl, 3,4,5-trifluorophenyl, 2,4,6-trifluorophenyl, 2,3,4,5-tetrafluorophenyl, 2,3,4,6-tetrafluorophenyl, or 2,3,5,6-tetrafluorophenyl group;
R5 is independently selected from a hydrogen atom, Boc, Fmoc or Z-group, or a label, wherein the label is selected from the group consisting of a biotin that is linked to the amino group via a spacer comprising -NH-C₁₋₂₀ alkyl-C(=0)-, NH-C₁₋₂₀ alkyl-C(=O)-NH-C₁₋₂₀ alkyl-C(=O)-, -NH-CH₂-CH₂-(O-CH₂-CH₂)ₙ-C(=O)- wherein n=1 to 6, or a random co-polymer of -NH-C₁₋₂₀ alkyl-C(=0)- and -NH-CH₂-CH₂-(O-CH₂-CH₂)ₙ-C(=O)-connected via amide bonds, wherein C₁₋₂₀ alkyl is branched or unbranched; a fluorescing moiety such as fluorescein, Cy5, Cy5.5, Sulfo-Cy5 or BODIPY; digoxygenin; a radioactive moiety; or an immunohistochemically detectable moiety; or R5 is a click-reactive functional group selected from the group comprising an alkyne, cyclooctyne derivative, azide derivative, alkene, hydrazine, hydrazine derivative, alkoxyamine, alkoxyamine derivative, aminoxy and thiol group;
with the proviso that, when R5 is a fluorescing moiety, then R2 is a C₆₋₁₁ aryl that may be unsubstituted or substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher, and R1 is a C₁₋₂₀ alkyl or C₃₋₂₀ cycloalkyl group, preferably methyl, ethyl, propyl or isopropyl;
with the proviso that, when R2 is a C₆₋₁₁ aryl that may be unsubstituted or substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher, then R5 is a fluorescing moiety and R1 is a C₁₋₂₀ alkyl or C₃₋₂₀ cycloalkyl group, preferably methyl, ethyl, propyl or isopropyl;
or pharmaceutically acceptable salt, isomer, enantiomer, tautomer or racemic mixture thereof.

Compounds of the invention can be synthesized as shown in the schematic diagram below. More detailed information on the specific reaction steps can be found in the Examples section herein.

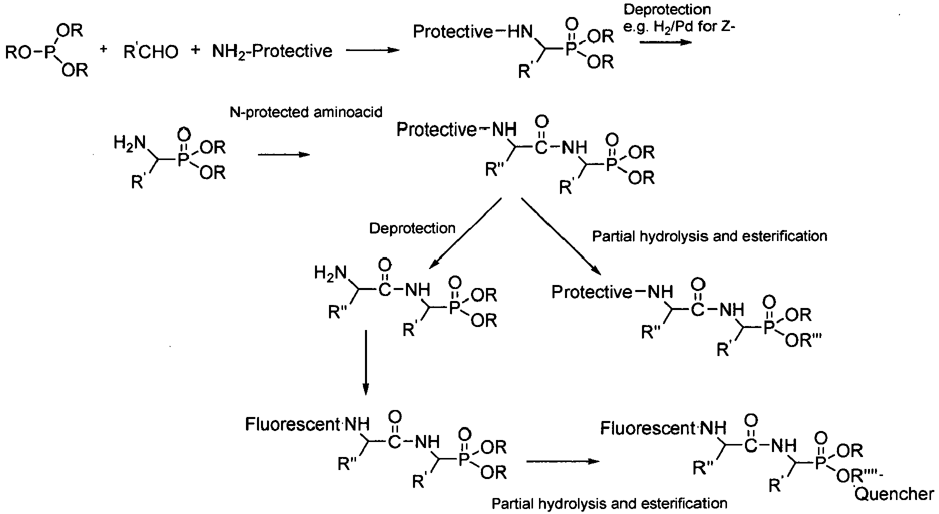

The compounds of the invention inhibit the activity of the serine protease KLK7. Accordingly, the compounds of the invention (or pharmaceutically acceptable salts, isomers, enantiomers, tautomers or racemic mixtures thereof) can be used for the treatment or prevention of diseases and disorders in a mammal, for example a human subject, for which the inhibition of KLK7 in the subject would be therapeutically effective. For example, the compounds of the invention are useful for the treatment of diseases or disorders in a mammal (e.g., human subject) associated with over-expression or undesired activity of KLK7. In one embodiment, the compounds of the present invention are suitable for the treatment or prevention of a skin disorder characterized by abnormal desquamation and/or inflammation. Said skin diseases or disorders include, but are not limited to, Netherton syndrome or other ichthyotic skin disorders such as autosomal dominant ichthyosis vulgaris, X-linked ichthyosis, lamellar ichthyosis or epidermolytic hyperkeratosis, as well as atopic dermatitis, psoriasis, acne rosacea, and palmoplantar keratosis to name a few. Preferably the skin disease or syndrome is Netherton syndrome.

In another embodiment, the compounds of the present invention are suitable for the treatment or prevention of an inflammatory disease. Preferably, the inflammatory disease is selected from the group comprising asthma, atherothrombosis, fibrotic and inflammatory kidney diseases, diabetes, chronic obstructive pulmonary disease and autoimmune diseases such as lupus nephritis and rheumatoid arthritis, among others.

In yet another embodiment, the compounds of the present invention are suitable for the treatment or prevention of a neurodegenerative disease such as amyotrophic lateral sclerosis, Parkinson's disease, Alzheimer's, Huntington's disease and ataxia telangiectasia.

In yet another embodiment, the compounds of the present invention are suitable for the treatment or prevention of cancer. In preferred embodiments, cancer is selected from the group comprising ovarian cancer, pancreatic cancer, melanoma, cervical cancer, papillary thyroid cancer, gliomas, oral squamous cell carcinoma, prostate cancer, renal cell carcinoma and colon cancer.

The invention also provides for compositions and medicaments comprising a compound of Formula I and at least one pharmaceutically acceptable carrier, auxiliary substance or excipient. These substances that can be added to therapeutic agents are known to the expert and include preservatives, wetting or emulsifying agents, pH buffering agents, stabilizers, detergents, antioxidants, carriers, flavoring agents, phospholipids, fillers, disintegrants, binders, lubricants, wetting agents, stabilizers, sweeteners, colorants, thickening agents, solvents, solubilizers, agents for generating sustained release tablets, salts for varying the osmotic pressure, coating agents, and other factors.

The pharmacological form of the particular composition will generally depend on the method by which the composition is administered to a subject. The compositions described herein are formulated for administration to a subject via any conventional means including, but not limited to, parenteral, transdermal, oral, topical administration, ophthalmic, otic or rectal administration routes. The term "parenteral" as used herein includes subcutaneous, intravenous, intraarterial, intramuscular, intraperitoneal, intrathecal, intraventricular, intrasternal, intracranial, and intraosseous injection and infusion techniques. For instance, the pharmaceuticals may be administered parenterally, for example intravenously, intramuscularly or subcutaneously, in the form of injection solutions or infusion solutions, microcapsules, or implants. Administration, however, can also be performed orally, for example in the form of pills, tablets, lacquered tablets, coated tablets, granules, hard and soft gelatin capsules, solutions, syrups, emulsions, suspensions or aerosol mixtures, or transdermally, or locally, for example in the form of ointments, solutions or tinctures, or rectally, for example in the form of suppositories, or in other ways, for example in the form of aerosols or nasal sprays.

Parenteral dosage forms may be administered in the form of sterile or sterilizable injectable preparations such as injectable solutions, suspensions, dry and/or lyophylized products ready to be dissolved or suspended in a pharmaceutically acceptable vehicle for injection (reconstitutable powders) and emulsions. Among the acceptable vehicles and solvents that can be employed are water, dextrose, Ringer's solution and isotonic sodium chloride solution, water-miscible vehicles such as, but not limited to, ethyl alcohol, polyethylene glycol, and polypropylene glycol; and non-aqueous vehicles such as, but not limited to, vegetable oils (e.g. corn oil, cottonseed oil, sesame oil), injectable organic esters such as ethyl oleate and isopropyl myristate, and benzyl benzoate.

Typical transdermal formulations comprise a conventional aqueous or non-aqueous vehicle, for example a cream, ointment, lotion or paste or are in the form of a medicated plaster, patch or membrane. Bases for such formulations include for example alcohols, lanolin, petrolatum, paraffin, polyethylene glycol, emulsifiers, penetration enhancing agents, and oleaginous vehicles such as oils. The transdermal delivery systems may include penetration enhancers to assist in delivering one or more compounds of Formula (I) to the tissue. Such penetration enhancers include, but are not limited to, acetone; various alcohols such as ethanol, oleyl, and tetrahydrofuryl; urea; alkyl sulfoxides such as dimethyl sulfoxide; pyrrolidones such as polyvinylpyrrolidone; dimethyl acetamide; dimethyl formamide; polyethylene glycol; Kollidon (Povidone, Polyvidone); and various water-soluble or insoluble sugar esters such as polysorbate 80 and sorbitan monostearate. Skin patches may also be used, typically consisting of a fabric or paper base impregnated with a suitable dose in a transdermal formulation.

Formulations for oral administration in solid or liquid form can be prepared according to any method known in the manufacture of pharmaceutical compositions and such compositions may contain one or more agents selected from the group comprising sweetening agents, flavoring agents and colorants in order to provide palatable preparations. Other useful excipients may be for example, inert diluents such as calcium carbonate, sodium carbonate, lactose, calcium phosphate or sodium phosphate, granulating and disintegrating agents, as well as other non-toxic compatible fillers, binders, non-ionic, cationic and anionic surfactants, buffers, antioxidants, lubricants, preservatives or stabilizers, and the like commonly used in pharmaceutical formulations. The tablets may be uncoated or may be coated by known techniques. In some cases such coatings can be prepared by known techniques to delay disintegration and absorption in the gastrointestinal tract and thereby provide a sustained action over a longer period. For example, a time delay material may be used such as monoglycerides or diglycerides of stearic acid. Suitable excipients for the production of solutions, for example injection solutions, or of emulsions or syrups are, for example, water, saline, alcohols, glycerol, polyols, sucrose, invert sugar, glucose, vegetable oils, and others. Suitable excipients for microcapsules or implants are, for example, copolymers of glycolic acid and lactic acid.

For rectal administration in the form of suppositories, the compositions can be prepared by mixing the drug with a suitable non-irritating excipient such as cocoa butter, synthetic glyceride esters or polyethylene glycols.

The pharmaceutical preparations normally contain about 0.5% to 90% by weight of the compounds of formula (I) and/or their pharmacologically acceptable salts. The amount of the active ingredient of formula (I) and/or their pharmacologically acceptable salts in the pharmaceutical preparations normally is from about 0.5 mg to about 1000 mg, preferably from about 1 mg to about 500 mg.

The pharmaceutical composition is administered to the subject in a therapeutically effective dose or a prophylactically effective dose. A "therapeutically effective dose" of a pharmaceutical composition is an amount sufficient to stop, reverse or reduce the progression of a disorder. A "prophylactically effective dose" of a pharmaceutical composition is an amount sufficient to prevent a disorder, i.e., eliminate, ameliorate and/or delay the disorder's onset. The dose can vary within wide limits and, as is customary and known to the physician, will be adapted to the specific conditions in each individual case. The dose depends, for example, on the specific compound employed, on the nature and severity of the disease to be treated, the manner and schedule of administration, or whether it is used for acute or chronic disease or as prophylaxis. The appropriate dosage can be established using clinical approaches well known in the medical art. In general, the daily dose for achieving the desired results in an adult weighing about 75kg is from 0.01 mg/kg to 100mg/kg, preferably from 0.1mg/kg to 50 mg/kg, in particular from 0.1 mg/kg to 10 mg/kg (in each case in mg per kg of body weight). The daily dose can be divided, in particular in the case of administration of relatively large amounts, into several, for example 2, 3 or 4 individual administrations. As usual, depending on the response of the individual subject it may be necessary to adjust upwards or downwards the indicated daily dose.

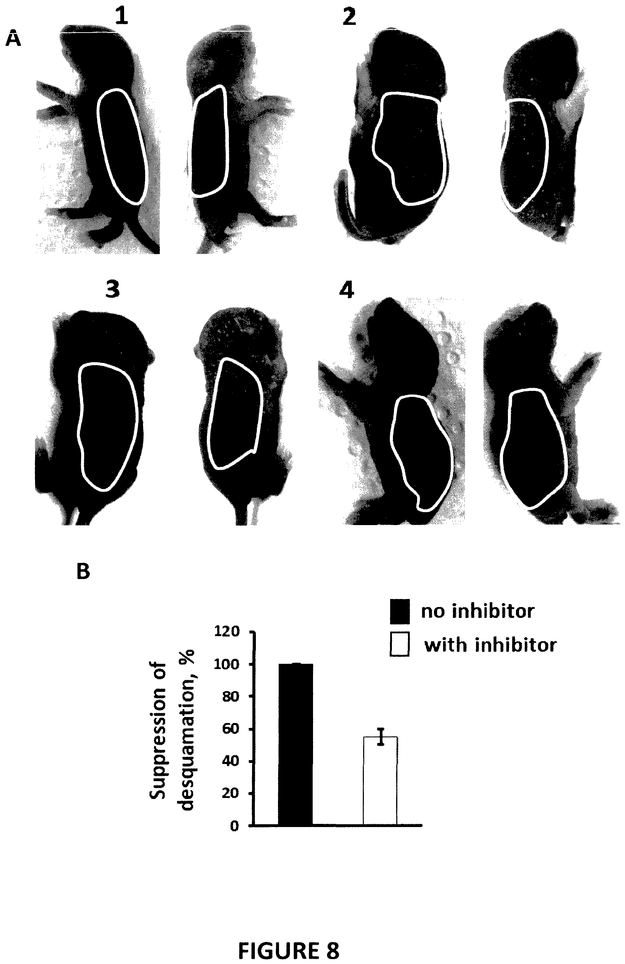

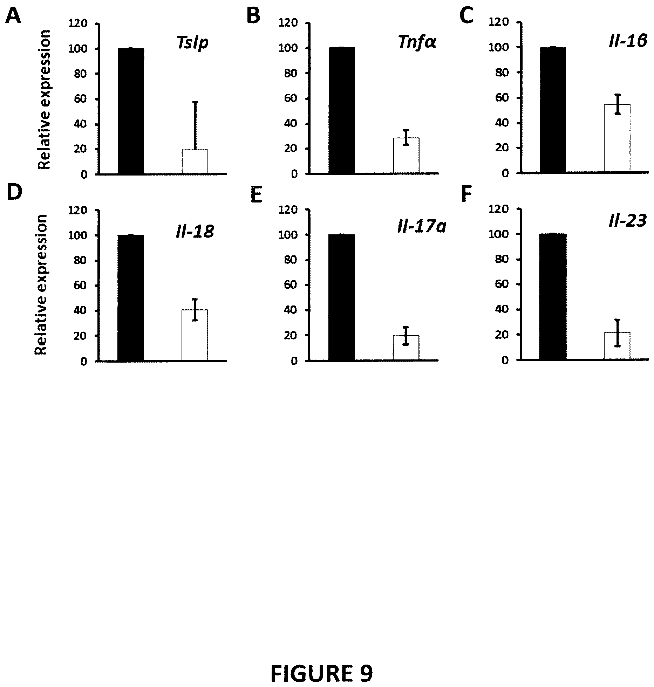

In one embodiment, the compounds or pharmaceutical compositions of the present invention can be used in combination with a KLK5 inhibitor to treat or prevent diseases or disorders in a mammal, for example in humans, for which the simultaneous inhibition of KLK7 and KLK5 in a patient will have therapeutic effect. This becomes particularly understood by observing Figures 8 and 9 where there is a significant improvement in the symptoms of Netherton's Syndrome in the Spink5^{-/-} mouse carrying a deactivated Klk5 gene (Klk5 targeting) after administration of the KLK7/Klk7 inhibitor. Thus, the compounds of the invention in combination with a KLK5 inhibitor are useful for the treatment of diseases or disorders in a mammal (e.g. human) that are associated with overexpression or unwanted activity of KLK7 and KLK5. In one embodiment, the compounds of the present invention in combination with a KLK5 inhibitor are suitable for the treatment or prevention of a skin disease characterized by abnormal exfoliation and / or inflammation such as Netherton Syndrome and atopic dermatitis (since Spink5^{-/-} is also a model of atopic dermatitis) or other ailments; of an inflammatory disease such as asthma, atherosclerosis, fibrotic and inflammatory kidney disease, diabetes, chronic obstructive pulmonary disease and autoimmune diseases such as neurodegenerative disease lateral sclerosis, Parkinson's disease, Alzheimer's disease, Huntington's disease and ataxia-telangiectasia among others; or cancer such as ovarian cancer, pancreatic cancer, melanoma, cervical cancer, cervical cancer, thyroid, gliomas, squamous cell carcinoma of the mouth, prostate cancer, renal cell carcinoma, and colorectal cancer.

The inhibitors of KLK7 and KLK5 can be administered simultaneously or sequentially according to a prescribed administration protocol, or formulated as a composition, that is, as a fixed dose combination.

When administered to the subject for the treatment of the diseases or syndromes disclosed herein, certain compounds of the invention can be further used for obtaining an in vivo image thereof. Thus, such compounds have a dual use after administration to the subject: (a) in therapy and (b) in a method of obtaining an in vivo image of said compound in the cell or tissue that is being treated. The method of in vivo imaging may be useful for assessing the uptake of said compound by the diseased cell or tissue that is being treated, determining the effective dose and frequency of administration, the responsiveness to therapy and/or the likelihood that the cell or tissue will respond to therapy. Specifically, compounds of formula (I) wherein:
R1 and R2 are independently selected from branched or unbranched: C₁₋₆alkyl; heteroalkyl; C₂₋₇ alkenyl; C₂₋₇ alkynyl; C₆₋₁₁ cycloalkyl; heterocyclyl; C₆₋₁₁ aryl; C₆₋₁₁ aryl-C₁₋₆ alkyl; heteroaryl; heteroaryl-C₁₋₆ alkyl; C₁₋₆ alkoxy; heterocyclyloxy; C₂₋₇ acyl; C₂₋₇ acyloxy; C₁₋₆ alkoxycarbonyl; naphthyl; or anthracenyl group; wherein each may be unsubstituted or substituted by one or more of halogen, hydroxy, amino, aminoalkyl, thiol, cyano, nitro, oxo, carboxy, C₁₋₃ alkyl, carboxyalkyl or C₁₋₃ alkoxy group; Preferably, R1 and R2 are independently selected from branched or unbranched: C₁₋₆ alkyl, C₅₋₁₁ cycloalkyl, phenyl, phenyl-C₁₋₆ alkyl, C₁₋₆ alkoxy, C₁₋₆ acyl, C₂₋₆ acyloxy or C₁₋₆ alkoxycarbonyl group, wherein each may be unsubstituted or substituted by one or more of halogen, hydroxy, amino, nitro, oxo, carboxy, C₁₋₃ alkyl, carboxyalkyl or C₁₋₃ alkoxy group. Most preferably, R1 and R2 are independently selected from phenyl, nitrophenyl, chlorophenyl, dinitrophenyl, dichlorophenyl, difluoroethyl or trifluoroethyl group. Alternatively, and in the embodiments where R5 is a fluorescing moiety, then necessarily R1 is C₁₋₂₀ alkyl or C₃₋₂₀ cycloalkyl group, preferably methyl, ethyl, propyl or isopropyl and R2 is a C₆₋₁₁ aryl that may be unsubstituted or substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher;
R3 and R4 are as defined herein;
R5 is a label selected from the group comprising a biotin that is linked to the amino group via a spacer comprising -NH-C₁₋₂₀ alkyl-C(=0)-, NH-C₁₋₂₀ alkyl-C(=O)-NH-C₁₋₂₀alkyl-C(=O)-, -NH-CH₂-CH₂-(O-CH₂-CH₂)ₙ-C(=O)- wherein n=1 to 6, or a random co-polymer of -NH-C₁₋₂₀ alkyl-C(=0)- and -NH-CH₂-CH₂-(O-CH₂-CH₂)ₙ-C(=O)- connected via amide bonds, wherein C₁₋₂₀ alkyl is branched or unbranched; a fluorescing moiety such as fluorescein, Cy5, Cy5.5, Sulfo-Cy5 or BODIPY; digoxygenin; a radioactive moiety; or an immunohistochemically detectable moiety, or R5 is a click-reactive functional group selected from the group comprising an alkyne, cyclooctyne derivative, azide derivative, alkene, hydrazine, hydrazine derivative, alkoxyamine, alkoxyamine derivative, aminoxy and thiol group;
or pharmaceutically acceptable salt, isomer, enantiomer, tautomer or racemic mixture thereof, can be used for in vivo imaging of said compounds after therapeutic administration to the subject.

The detectable label can include optically detectable moieties, such as a fluorophore, chromophore, luminophore, quantum dot or nanoparticle light scattering label; electromagnetic spin label; calorimetric agent; magnetic substance; electron-rich material such as a metal; electrochemiluminescent label; paramagnetic moiety; light scattering or plasmon resonant materials, such as gold or silver particles; or multielement reporter systems, such as affinity tags including but not limited to enzyme and substrate reporter groups. Suitable fluorophores include Alexa Fluor dyes, BODIPY, Texas Red, Coumarin, Pacific Green, Pacific Orange, Pacific Blue, Cy3, Cy5, rhodamine, tetramethylrhodamine (TRITC), Fluorescein (FITC) and Qdot dyes, among others. Isotopically labeled compounds of the present inventions labeled with a fluorophore can generally be prepared by starting with isotopically labeled reactants. Alternatively, compounds could be directly coupled with the isotopes as in the case of iodine (¹²³I or ¹²⁵I).

A biotin (5-[(3aS,4S,6aR)-2-oxohexahydro-1H-thieno[3,4-d]imidazol-4-yl]pentanoic acid) or imino biotin compound or amino-terminated derivative of biotin compound or carboxy-terminated derivative of biotin compound may be directly coupled with amine or hydroxy groups of the polymer and agent through the use of amide bond or ester bond forming coupling reagents, respectively. It may also be coupled through a spacer group such as -NH-C₁₋₂₀ alkyl-C(=0)-, NH-C₁₋₂₀ alkyl-C(=O)-NH-C₁₋₂₀ alkyl-C(=O)-, -NH-CH₂-CH₂-(O-CH₂-CH₂)ₙ-C(=O)- wherein n=1 to 6, or a random co-polymer of -NH-C₁₋₂₀alkyl-C(=0)- and -NH-CH₂-CH₂-(O-CH₂-CH₂)ₙ-C(=O)- connected via amide bonds, where C₁₋₂₀ alkyl in these examples is meant as branched or unbranched. The term "random copolymer" in this context refers to copolymers of the -NH-C₁₋₂₀ alkyl-C(=0)-group and the -NH-CH₂-CH₂-(O-CH₂-CH₂)ₙ-C(=O)- group, having two or more repetitive units which do not form an identical repeating pattern.

Exemplary radioactive moieties (isotopes) that can be incorporated in to compounds of the invention include isotopes of hydrogen, carbon, nitrogen, oxygen, phosphorous, sulfur, fluorine, chlorine and iodine, such as ²H ("D"), ³H, ¹¹C, ¹³C, ¹⁴C, ¹³N, ¹⁵N, ¹⁵O, ¹⁷O, ¹⁸O, ³²P, ³³P, ³⁵S, ¹⁸F, ³⁶Cl, ¹²³I and ¹²⁵I. Certain isotopically labeled compounds of the present invention (e.g., those labeled with ³H or ¹⁴C) are useful in compound tissue distribution assays. Tritiated (³H) and carbon-14 (¹⁴C) isotopes are useful for their ease of preparation and detectability. Further substitution with heavier isotopes such as deuterium (i.e., ²H) may afford certain therapeutic advantages resulting from greater metabolic stability (e.g., increased in vivo half-life or reduced dosage requirements) and hence may be preferred in certain applications. Positron emitting isotopes such as ¹⁵O, ¹³N, ¹¹C, and ¹⁸F are useful for positron emission tomography (PET) studies to examine tissue localization of the active KLK7 enzyme.

Paramagnetic substances or magnetic particles exhibiting ferromagnetic, ferrimagnetic or superparamagnetic behaviour are suitable agents for in vivo imaging with nuclear magnetic resonance. Paramagnetic MRI contrast agents can for example be transition metal chelates and lanthanide chelates like Mn EDTA (Manganese EDTA Chelate) and gadolinium-DTPA (diethylenetriamine penta-acetic acid). Several gadolinium based agents are in clinical use, including for example Magnevist®, Omniscan® and others. Magnetic particles proposed for use as MR contrast agents are water-insoluble substances such as Fe₃O₄ or O-Fe₂O₃ optionally provided with a coating or carrier matrix.

The in vivo detection and localization of the imaging agent may be achieved by radionuclide imaging, radioscintigraphy, nuclear magnetic resonance imaging, computed tomography, positron emission tomography, computerized axial tomography, X-ray or magnetic resonance imaging method, fluorescence detection, and chemiluminescent detection, depending on the particular label used.

In one preferred embodiment, the compounds according to the invention for dual use in therapy and in vivo imaging carry a fluorescing moiety such as fluorescein, Cy5, Cy5.5, Sulfo-Cy5 or BODIPY or any of the other fluorophores mentioned herein. The major limitation of compounds carrying a fluorescing moiety is that they fluoresce both when bound to an enzyme target and when free in solution. To overcome this limitation, the compounds disclosed herein also carry a fluorescent quencher. Thus the compounds of this preferred embodiment become fluorescent only after covalent modification of the enzyme target that releases the quencher but leaves the fluorescently labeled compound covalently attached to the enzyme. Suitable quenchers are DABCYL and TAMRA, the QSY® series and the Black Hole Quenchers (BHQs). The method of obtaining an in vivo image of said compound after administration to the subject may comprise the steps of (a) allowing the compound to distribute within the subject after administration, and (b) detecting the label using a whole body fluorescence monitoring device. The in vivo fluorescence may be determined with an in vivo imaging apparatus capable of recording the fluorescence in a spatial resolution manner.

In another preferred embodiment, the compounds for dual use in therapy and in vivo imaging carry a click-reactive functional group instead of a detectable label. In this case, the detectable label is carried on a second compound which comprises a click-reactive functional group that is complementary to the R5 click-reactive functional group. For example, the click-reactive functional group on a compound claimed herein may be a 1-alkyne group and the complementary click-reactive functional group on the second molecule may be an azide. In this way the inhibitor is a smaller molecule compared to the compounds carrying both inhibitory domain and detection tag and thus could have improved pharmacokinetic potential. The method of obtaining an in vivo image of said compound after administration to the subject may comprise the steps of (a) allowing the compound to distribute within the subject after administration, (a') administering to the subject a second compound comprising a click-reactive functional group that is complementary to the R5 click-reactive functional group, wherein said second compound further comprises a detectable label selected from the group comprising biotin, a fluorescing moiety, digoxygenin, a paramagnetic substance or magnetic particle, an electron dense reagent, a radioactive moiety and an immunohistochemically detectable moiety, (b) detecting the label using the appropriate imaging method and (c) correlating the presence, the levels, distribution and/or localization of said label to disease status.

In yet another preferred embodiment, the compounds for dual use in therapy and in vivo imaging carry a biotin that is linked to the amino group via a spacer as described herein. The method of obtaining an in vivo image of said compound after administration to the subject may comprise the steps of (a) allowing the compound to distribute within the subject after administration, (a') administering to the subject a second compound comprising streptavidin carrying a fluorescing moiety, (b) detecting the label using a whole body fluorescence monitoring device and (c) correlating the presence, the levels, distribution and/or localization of said label to disease status.

The compounds of the invention are particularly suitable for cosmetic use. Preferably, said compounds are used for the improvement of an undesirable skin condition. Said undesirable skin condition may include, without being limited to, eczema, such as seborrheic eczema, skin rashes, dry or scaly skin to name a few. According to this embodiment, the compositions may be formulated into all the pharmaceutical forms normally employed for topical application, in particular in the form of aqueous, aqueous/alcoholic or oily solutions, suspensions or dispersions of the lotion or serum type, anhydrous or lipophilic gels, emulsions of liquid or semi-liquid consistency of the milk type, obtained by dispersion of a fatty phase in an aqueous phase (oil-in-water) or conversely (water-in-oil), or suspensions or emulsions of smooth, semi-solid or solid consistency of the cream or gel type, or alternatively microemulsions, microcapsules or microparticles, or vesicle dispersions of ionic and/or nonionic type. These compositions are formulated according to conventional techniques.

The compounds may also be used for the hair in the form of aqueous, alcoholic or aqueous/alcoholic solutions, or in the form of creams, gels, emulsions or mousses or alternatively in the form of aerosol compositions also containing a propellant under pressure.

The cosmetic compositions of the invention may also contain additives and adjuvants which are common in the cosmetics, pharmaceutical or dermatological field, such as hydrophilic or lipophilic gelling agents, preservatives, antioxidants, solvents, fragrances, fillers, screening agents, bactericides, odor absorbers and dyestuffs and colorants. The amounts of these various additives and adjuvants are those used conventionally in the cosmetics field. The cosmetic compositions may also comprise one or more additional active agents. Examples of suitable active agents are antibacterial agents such as tetracyclin antibiotics, erythromycin and clindamycin, steroidal anti-inflammatory agents such as hydrocortisone and betamethasone, non-steroidal anti-inflammatory agents such as acetylsalicylic acid, ibuprofen, acetaminophen, or diclofenac, antipruriginous agents such as trimeprazine or cyproheptadine, keratolytic agents such as salicylic acid, citric acid or resorcinol; retinoids such as retinoic acid and retinol, and other agents.

In a preferred embodiment, the concentration of the one or more compounds according to the invention is between 0.001% and 10% by weight relative to the whole composition. Preferably, said concentration is between 0.01% and 4%, most preferably from 0.1% to 2% by weight relative to the whole composition.

In another aspect, the invention provides an in vitro method for the classification, diagnosis or prognosis of a disease with a KLK7 involvement, preferably for a skin disorder characterized by abnormal desquamation and/or inflammation such as Netherton syndrome or other ichthyotic skin disorders such as autosomal dominant ichthyosis vulgaris, X-linked ichthyosis, lamellar ichthyosis or epidermolytic hyperkeratosis, as well as atopic dermatitis, psoriasis, acne rosacea, palmoplantar keratosis among others; an inflammatory disease such as asthma, atherothrombosis, fibrotic and inflammatory kidney diseases, diabetes, chronic obstructive pulmonary disease and autoimmune diseases such as lupus nephritis and rheumatoid arthritis, among others; a neurodegenerative disease such as amyotrophic lateral sclerosis, Parkinson's disease, Alzheimer's disease, Huntington's disease and ataxia telangiectasia among others; or cancer such as ovarian cancer, pancreatic cancer, melanoma, cervical cancer, papillary thyroid cancer, gliomas, oral squamous cell carcinoma, prostate cancer, renal cell carcinoma and colon cancer.

The sample may be a cell, including tissue culture cell or cell derived there from and the progeny thereof, or a circulating tumor cell; cell extract; cell supernatant; tissue sample including a biopsy specimen, tissue section or other; bodily fluid such as blood, lymph, urine, amniotic fluid, vitreous fluid and cerebrospinal fluid (CSF); tissue homogenate or other.

The in vitro method claimed herein comprises the steps of (a) contacting the sample with a compound of formula (I) suitable for in vitro imaging, carrying a detectable label, (b) determining one or more parameters such as the presence, the levels, distribution and/or localization of said label and (c) correlating said one or more parameters to disease status. Said compound of formula (I) suitable for in vitro imaging is a compound wherein R1 and R2 are independently selected from branched or unbranched: C₁₋₆alkyl; heteroalkyl; C₂₋₇ alkenyl; C₂₋₇ alkynyl; C₆₋₁₁ cycloalkyl; heterocyclyl; C₆₋₁₁ aryl; C₆₋₁₁ aryl-C₁₋₆ alkyl; heteroaryl; heteroaryl-C₁₋₆ alkyl; C₁₋₆ alkoxy; heterocyclyloxy; C₂₋₇ acyl; C₂₋₇ acyloxy; C₁₋₆ alkoxycarbonyl; naphthyl; or anthracenyl group; wherein each may be unsubstituted or substituted by one or more of halogen, hydroxy, amino, aminoalkyl, thiol, cyano, nitro, oxo, carboxy, C₁₋₃ alkyl, carboxyalkyl or C₁₋₃ alkoxy group; Preferably, R1 and R2 are independently selected from branched or unbranched: C₁₋₆ alkyl, C₅₋₁₁ cycloalkyl, phenyl, phenyl-C₁₋₆ alkyl, C₁₋₆ alkoxy, C₁₋₆ acyl, C₂₋₆ acyloxy or C₁₋₆ alkoxycarbonyl group, wherein each may be unsubstituted or substituted by one or more of halogen, hydroxy, amino, nitro, oxo, carboxy, C₁₋₃ alkyl, carboxyalkyl or C₁₋₃ alkoxy group. Most preferably, R1 and R2 are independently selected from phenyl, nitrophenyl, chlorophenyl, dinitrophenyl, dichlorophenyl, difluoroethyl or trifluoroethyl group. Alternatively, in certain embodiments where R5 is a fluorescing moiety, then R1 may be a C₁₋₂₀ alkyl or C₃₋₂₀ cycloalkyl group, preferably methyl, ethyl, propyl or isopropyl and R2 may be a C₆₋₁₁ aryl that may be unsubstituted or substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher;
R3 and R4 are as defined herein;
R5 is a label selected from the group comprising a biotin that is linked to the amino group via a spacer comprising -NH-C₁₋₂₀ alkyl-C(=0)-, NH-C₁₋₂₀ alkyl-C(=O)-NH-C₁₋₂₀ alkyl-C(=O)-, -NH-CH₂-CH₂-(O-CH₂-CH₂)ₙ-C(=O)- wherein n=1 to 6, or a random co-polymer of -NH-C₁₋₂₀ alkyl-C(=0)- and -NH-CH₂-CH₂-(O-CH₂-CH₂)ₙ-C(=O)- connected via amide bonds, wherein C₁₋₂₀ alkyl is branched or unbranched; a fluorescing moiety such as fluorescein, Cy5, Cy5.5, Sulfo-Cy5 or BODIPY; digoxygenin; a radioactive moiety; or an immunohistochemically detectable moiety;
or salt, isomer, enantiomer, tautomer or racemic mixture thereof.

The reference sample may be a tissue or fluid or other biological sample from a subject without the disease, such as a corresponding tissue, fluid or other sample, such as a normal blood sample, a normal tissue sample, or a normal cell homogenate. Alternatively, the reference sample may be obtained from the subject, but from a tissue or fluid not expected to contain active KLK7; such samples may be used as reference or normal samples.

In some cases, statistical analysis is performed to determine whether there is a difference, such as a significant difference, between the levels of label detected in the test biological sample and the reference sample. For example, a difference may be considered significant where there is a p value of less than 0.05 or where there is a ± 2 standard deviation. Other methods for determining significance are known in the art.

As already discussed herein, over-expression, or ectopic expression or activation or KLK7 is implicated in disease progression in Netherton syndrome, atopic dermatitis, ovarian cancer, pancreatic cancer, melanoma, cervical cancer, papillary thyroid cancer, gliomas, oral squamous cell carcinoma, prostate cancer, renal cell carcinoma and colon cancer among others. Conversely, the down-regulation of KLK7 in Alzheimer's disease correlates with disease progression. For instance, reduced levels of active KLK7 in a particular sample (e.g. blood, plasma, serum, CSF) may be an indication that the subject has or may develop Alzheimer's disease. Similarly, information on the classification of disease may be obtained from measuring the levels of active KLK7 in a particular diseased tissue; for instance, different KLK7 levels may discriminate between low grade and high grade clear cell renal cell carcinoma (RCC) and can also distinguish between oncocytoma and chromophobe RCC.

In certain embodiments, the step of determining the presence, the amount and/or localization of said detectable label comprises Enzyme-Linked Immunosorbent Assay (ELISA), histochemistry, Western-like blotting, immunoprecipitation, gel zymography, immunohistochemistry, fluorescence microscopy, flow cytometry, fluorescence in situ hybridization (FISH) or a radioimmunoassay. For instance, when R5 is a fluorescing moiety such as fluorescein, Cy5, Cy5.5, Sulfo-Cy5 or BODIPY, the detection method would be a fluorescence-based method such as fluorometry or fluorescence microscopy.

In a preferred embodiment, the in vitro method for measuring the presence and/or levels of the detectable label (and thus the levels of active KLK7) in a sample is an ELISA assay. Example 5 describes the development of a sandwich-like ELISA assay to determine the levels of active KLK7 in biological or clinical samples. Specifically, the procedure includes the incubation of ABP with the biological/clinical specimen to be tested and then addition of this mixture to an ELISA plate with immobilized anti-KLK7 antibody. The KLK7-ABP adduct is captured by the immobilized antibody and is detected by a typical ELISA detection method e.g. through horseradish peroxidase assisted reaction. Alternative ELISA formats to improve the detection limit could incorporate other systems of detection e.g. fluorescence such as time resolved fluorescence. Alternatively, the KLK7-ABP could be immobilized on the ELISA plate and the antibody could be used for detection.

In an alternative embodiment, the in vitro method disclosed herein is used for monitoring response to a medical treatment. Said method comprises the steps of (a) contacting a sample (a cell extract, cell, tissue sample including tissue biopsy and tissue section, bodily fluid, tissue homogenate or other) obtained from the subject after treatment with a particular medication, with the compound of formula (I) suitable for in vitro imaging as defined above, (b) determining the presence, the levels and/or localization of said label, (c) comparing the presence, the levels and/or localization of said label to a sample from the same subject prior to treatment, and (d) correlating the differences in the presence, the levels, distribution and/or localization of said label to response to treatment.

In a further aspect, certain compounds of the present invention are useful in a method for the in vivo imaging of a disease or disorder with a KLK7 involvement. Preferably, such a disease or disorder is a skin disorder characterized by abnormal desquamation and/or inflammation such as Netherton syndrome or other ichthyotic skin disorders such as autosomal dominant ichthyosis vulgaris, X-linked ichthyosis, lamellar ichthyosis or epidermolytic hyperkeratosis, as well as atopic dermatitis, psoriasis, acne rosacea, palmoplantar keratosis among others; an inflammatory disease such as asthma, atherothrombosis, fibrotic and inflammatory kidney diseases, diabetes, chronic obstructive pulmonary disease and autoimmune diseases such as lupus nephritis and rheumatoid arthritis, among others; a neurodegenerative disease such as amyotrophic lateral sclerosis, Parkinson's disease, Alzheimer's disease, Huntington's disease and ataxia telangiectasia among others; or cancer such as ovarian cancer, pancreatic cancer, melanoma, cervical cancer, papillary thyroid cancer, gliomas, oral squamous cell carcinoma, prostate cancer, renal cell carcinoma and colon cancer. Particularly, compounds of the invention useful for in vivo imaging are compounds of formula (I) wherein R1 and R2 are independently selected from branched or unbranched: C₁₋₆alkyl; heteroalkyl; C₂₋₇ alkenyl; C₂₋₇ alkynyl; C₆₋₁₁ cycloalkyl; heterocyclyl; C₆₋₁₁ aryl; C₆₋₁₁ aryl-C₁₋₆ alkyl; heteroaryl; heteroaryl-C₁₋₆ alkyl; C₁₋₆ alkoxy; heterocyclyloxy; C₂₋₇ acyl; C₂₋₇ acyloxy; C₁₋₆ alkoxycarbonyl; naphthyl; or anthracenyl group; wherein each may be unsubstituted or substituted by one or more of halogen, hydroxy, amino, aminoalkyl, thiol, cyano, nitro, oxo, carboxy, C₁₋₃ alkyl, carboxyalkyl or C₁₋₃ alkoxy group; Preferably, R1 and R2 are independently selected from branched or unbranched: C₁₋₆ alkyl, C₅₋₁₁ cycloalkyl, phenyl, phenyl-C₁₋₆ alkyl, C₁₋₆ alkoxy, C₁₋₆ acyl, C₂₋₆ acyloxy or C₁₋₆ alkoxycarbonyl group, wherein each may be unsubstituted or substituted by one or more of halogen, hydroxy, amino, nitro, oxo, carboxy, C₁₋₃ alkyl, carboxyalkyl or C₁₋₃ alkoxy group. Most preferably, R1 and R2 are independently selected from phenyl, nitrophenyl, chlorophenyl, dinitrophenyl, dichlorophenyl, difluoroethyl or trifluoroethyl group. Alternatively, and in the embodiments where R5 is a fluorescing moiety, then necessarily R1 is C₁₋₂₀ alkyl or C₃₋₂₀ cycloalkyl group, preferably methyl, ethyl, propyl or isopropyl and R2 is a C₆₋₁₁ aryl that may be unsubstituted or substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher;
R3 and R4 are as defined herein;
R5 is a label selected from the group comprising a biotin that is linked to the amino group via a spacer comprising -NH-C₁₋₂₀ alkyl-C(=0)-, NH-C₁₋₂₀alkyl-C(=O)-NH-C₁₋₂₀ alkyl-C(=O)-, -NH-CH₂-CH₂-(O-CH₂-CH₂)ₙ-C(=O)- wherein n=1 to 6, or a random co-polymer of -NH-C₁₋₂₀ alkyl-C(=0)- and -NH-CH₂-CH₂-(O-CH₂-CH₂)ₙ-C(=O)- connected via amide bonds, wherein C₁₋₂₀ alkyl is branched or unbranched; a fluorescing moiety such as fluorescein, Cy5, Cy5.5, Sulfo-Cy5 or BODIPY; digoxygenin; a radioactive moiety; or an immunohistochemically detectable moiety, or R5 is a click-reactive functional group selected from the group comprising an alkyne, cyclooctyne derivative, azide derivative, alkene, hydrazine, hydrazine derivative, alkoxyamine, alkoxyamine derivative, aminoxy and thiol group;
or pharmaceutically acceptable salt, isomer, enantiomer, tautomer or racemic mixture thereof.

The preferred method of in vivo imaging comprises the steps of (a) administering the compound to a subject, (b) allowing the compound to distribute within the subject, and (c) detecting the label; and wherein, when R5 is a click-reactive functional group, the method further comprises, after step (b), administering to the subject a second compound comprising a click-reactive functional group that is complementary to the R5 click-reactive functional group, and wherein the second compound further comprises a radioactive label, paramagnetic substance or magnetic particle, a fluorophore, an electron dense reagent, biotin or digoxigenin.

The claimed method for in vivo imaging is preferably for use in diagnosis, guided surgery, monitoring, assessment and/or staging of a disease or a condition and/or of a subject's response to a medical treatment. For instance, and as already discussed, the presence, levels or localization of the detectable label corresponds to the presence, levels or localization of activated KLK7. Thus, for example, increased levels of the detectable label in a melanoma could be related with more invasive forms. In skin disorders such as Netherton syndrome or atopic dermatitis, increased levels of detectable label could be related with more severe desquamating or inflammatory phenotype. Another application of the invention is in image-guided surgery such as image-guided laser assisted surgery for detection of micrometastases of tumours, tumour margins, or radioimmunoguided surgery. Once administered to a subject, the compounds allow for more successful surgical outcomes, for example due to the ability of a surgeon to identify those bodily fluids and organs which contain the administered compounds. Identification of these fluids and organs enables the surgeon to avoid unwanted damage to non-target organs and tissues during surgery. Yet another application is in monitoring a subject's response to a medical treatment. For example in Netherton syndrome higher dosage of the compounds could be required relative to atopic dermatitis for treatment. Thus higher levels of detectable label will be found in Netherton syndrome. This could help adjust the dose for each individual during treatment (personalized treatment).

The compositions are administered in doses effective to achieve the desired optical image of a tumour, tissue, or organ. Such doses may vary widely, depending upon the particular dye compound employed, the tissue, or organ subjected to the imaging procedure, the imaging equipment being used, and the like. For instance, when the label is a paramagnetic substance, higher dosages may be required than when the label is a radioactive moiety. The label can be detected using a method suitable for the individual label, said method may comprise fluorescent microscopy, optical imaging, near infrared (NIR) imaging, molecular fluorescence endoscopy, magnetic resonance spectroscopy (MRS) or imaging (MRI), or gamma imaging such as positron emission tomography (PET), or single photon emission computed tomography (SPECT).

In one preferred embodiment, the compound of formula (I) useful for in vivo imaging, carries a fluorescent label. Thus, R5 is a fluorescing moiety such as fluorescein, Cy5, Cy5.5, Sulfo-Cy5 or BODIPY; R1 is a C₁₋₂₀ alkyl or C₃₋₂₀ cycloalkyl group, preferably methyl, ethyl, propyl or isopropyl; R2 is a C₆₋₁₁ aryl that may or may not be substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher; preferably a phenyl, chlorophenyl or dichlorophenyl group, each further substituted with an amino, aminoalkyl(C₁₋₂₀), carboxy or carboxyalkyl(C₁₋₂₀) group covalently attached to a fluorescent quencher. In this preferred embodiment, the method of in vivo imaging comprises (a) administering the compound to a subject, (b) allowing the compound to distribute within the subject, and (c) detecting the label. The step of detecting the detectable label is achieved by a method selected from the group comprising fluorescent microscopy, optical imaging, near infrared (NIR) imaging and molecular fluorescence endoscopy.

In another preferred embodiment, the compound of formula (I) useful for in vivo imaging does not carry a detectable label, instead it carries a click-reactive functional group and can be indirectly labeled by reacting with a complementary click functional group carrying a detectable label. In this preferred embodiment, the method of in vivo imaging comprises (a) administering the compound to a subject, (b) allowing the compound to distribute within the subject, (b') administering to the subject a second compound comprising a click-reactive functional group that is complementary to the R5 click-reactive functional group and wherein the second compound further comprises a detectable label selected from the group comprising a radioactive label, a fluorophore, a paramagnetic substance or magnetic particle, an electron dense reagent, biotin or digoxigenin, and (c) detecting the label. The step of detecting the label is achieved by a method selected from the group comprising fluorescent microscopy, optical imaging, near infrared (NIR) imaging, molecular fluorescence endoscopy, magnetic resonance spectroscopy (MRS) or imaging (MRI), or gamma imaging such as positron emission tomography (PET), or single photon emission computed tomography (SPECT).

In yet another aspect, the invention provides kits for use in the treatment, prevention or diagnosis of a disease or disorder with a KLK7 involvement. Preferably, said disease or disorder is a skin disorder characterized by abnormal desquamation and/or inflammation such as Netherton syndrome or other ichthyotic skin disorders such as autosomal dominant ichthyosis vulgaris, X-linked ichthyosis, lamellar ichthyosis or epidermolytic hyperkeratosis, as well as atopic dermatitis, psoriasis, acne rosacea, palmoplantar keratosis among others; an inflammatory disease such as asthma, atherothrombosis, fibrotic and inflammatory kidney diseases, diabetes, chronic obstructive pulmonary disease and autoimmune diseases such as lupus nephritis and rheumatoid arthritis, among others; a neurodegenerative disease such as amyotrophic lateral sclerosis, Parkinson's disease, Alzheimer's disease, Huntington's disease and ataxia telangiectasia among others; or cancer such as ovarian cancer, pancreatic cancer, melanoma, cervical cancer, papillary thyroid cancer, gliomas, oral squamous cell carcinoma, prostate cancer, renal cell carcinoma and colon cancer. Said kits comprise (a) the compound of formula (I) having a KLK7 inhibitory activity, wherein R1 and R2 are independently selected from branched or unbranched: C₁₋₆alkyl, C₅₋₁₁ cycloalkyl, phenyl, phenyl-C₁₋₆ alkyl, C₁₋₆ alkoxy, C₁₋₆ acyl, C₂₋₆ acyloxy or C₁₋₆ alkoxycarbonyl group, wherein each may be unsubstituted or substituted by one or more of halogen, hydroxy, amino, nitro, oxo, carboxy, C₁₋₃ alkyl, carboxyalkyl or C₁₋₃ alkoxy group;
R3 and R4 are as defined herein;
R5 is a click-reactive functional group selected from the group comprising an alkyne, cyclooctyne derivative, azide derivative, alkene, hydrazine, hydrazine derivative, alkoxyamine, alkoxyamine derivative, aminoxy and thiol group;
or pharmaceutically acceptable salt, isomer, enantiomer, tautomer or racemic mixture thereof, in a suitable carrier; and
(b) a second compound comprising a click-reactive functional group that is complementary to the R5 click-reactive functional group and wherein said second compound further comprises a detectable label selected from the group comprising biotin, a fluorescing moiety, digoxygenin, a paramagnetic substance or magnetic particle, an electron dense reagent, a radioactive moiety, or an immunohistochemically detectable moiety, in a suitable carrier.

The kit may optionally comprise inactive agents or compounds or other items which are known in the art, such as an information leaflet, diluents, buffers, empty syringes, tubing, gauze pads, disinfectant solution, etc.

The first compound in a suitable carrier and the second compound in a suitable carrier may be formulated for e.g., parenteral administration, for example, injection or infusion, orally, by inhalation spray, rectally, nasally, buccally, vaginally, via an implanted reservoir or locally. The exact administration protocol will vary depending upon various factors including the age, body weight, general health, sex and diet of the patient; the determination of specific administration procedures would be routine to any one of ordinary skill in the art.

### EXAMPLES

Hereinafter, the present invention is described in more detail with reference to examples. It will be apparent to one of ordinary skill in the art that these examples are for illustrative purposes only and should not be construed as limiting or altering the scope of the present invention.

### EXAMPLE 1

### Synthesis of Inhibitor 1 (Boc-FFP; tert-butvl 1-(1-(diphenoxvphosphorvl)-2-phenylethylamino)-1-oxo-3-phenylpropan-2-ylcarbamate)

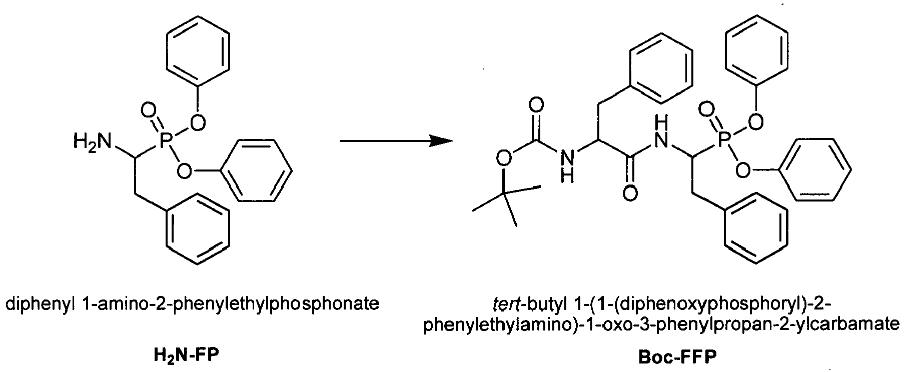

Diphenyl 1-amino-2-phenylethylphosphonate (H₂N-FP) was synthesized according to previously published method (Oleksyszyn et al., Synthesis, pp. 985-986, 1979). To a stirred solution of H₂N-FP (0.31 mmol, 110 mg) and tert-butyloxycarbonyl-L-phenylalanine (0.35 mmol, 93 mg) in dichloromethane (2 mL) were added N,N'-diisopropylethylamine (DIPEA, 0.93 mmol, 0.16 mL), 2-(1H-benzotriazol-1-yl)-1,1,3,3-tetramethyluronium tetrafluoroborate (TBTU, 0.35 mmol, 133 mg) and 1-hydroxybenzotriazole (HOBt, 0.35 mmol, 47 mg). The reaction was performed at room temperature overnight. DIPEA was added if necessary to maintain the alkaline pH. The reaction was monitored by TLC until H₂N-FP was consumed. Then the mixture was diluted with dichloromethane (15 mL) and washed sequentially with aqueous citric acid (10%, 20 mL x 3), aqueous NaHCO₃ (5%, 20 mL x 3), H₂O (20 mL) and brine (20 mL), dried (Na₂SO₄), filtered, and concentrated in vacuo to give, after purification by column chromatography (silica gel, Hexanes/Ethyl acetate 60:40), the desired Boc-protected phosphonic dipeptide Boc-FFP as a white solid (63 mg, yield 42%). ¹H-NMR (600 MHz, 298 K, CDCl3): δ 7.32-7.01 (m, 20H), 6.58 (m, 1H), 5.11 (m, 1H), 4.62 (m, 1H), 4.26 (m, 1H), 3.39-2.74 (m, 4H), 1.37 (s, 9H). ¹³C-NMR (151 MHz, 298 K, CDCl₃): δ 170.70, 150.30, 150.04, 149.95, 149.87, 133.61, 129.67, 129.52, 129.23, 129.09, 128.77, 128.76, 128.70, 128.57, 128.44, 128.23, 126.82, 125.30, 125.20, 125.16, 120.46, 120.44, 120.41, 120.27, 51.08, 51.03, 50.03, 49.98, 28.09. ³¹P-NMR (243 MHz, 298 K, CDCl₃): δ 16.51, 16.24. ESI-MS: m/z calcd for C₃₄H₃₇N₂O₆P [M + Na]⁺ 623.23; found 623.09.

### EXAMPLE 2

### Synthesis of Biotin-FFP (diphenyl 1-(2-(6-(6-(5-((4R)-2-oxo-hexahydro-1H-thieno[3,4-d]imidazole-4-yl)pentanamido)hexanamido)hexanamido)-3-phenylpropanamido)-2-phenylethylphosphonate)

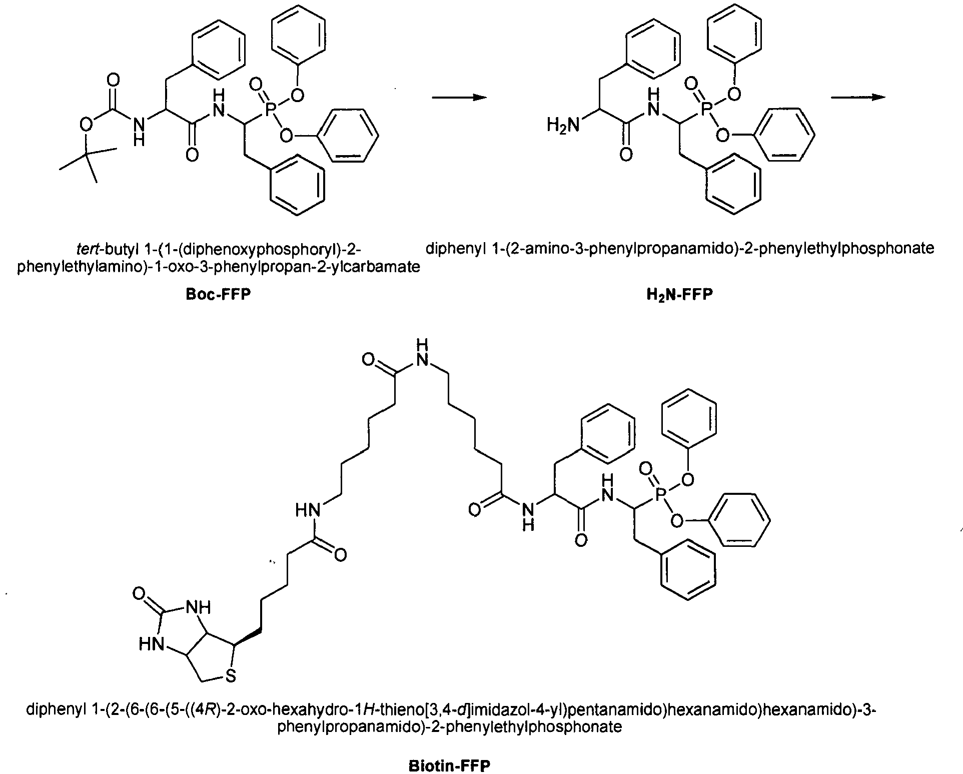

The **Boc-FFP** (0.017 mmol, 10 mg) was dissolved in dichloromethane (0.4 mL) and an equal volume of trifluoroacetic acid was added dropwise. The reaction was performed at room temperature and the progress was monitored with TLC. When reaction was completed (approx. 2h), the volatile components were removed under reduced pressure to give the unprotected phosphonic dipeptide **H₂N-FFP** as trifluoroacetate salt (10.25 mg, quant.). The product was checked with ESI-MS (*m*/*z* calculated for **H₂N-FFP** (C₂₉H₂₉N₂O₄P) [M + H]⁺ 501.19; found 501.12) and was used directly in the next step without further purification. The diphenyl 1-(2-(6-(6-(5-((3a*S*,4*R*,6a*R*)-2-oxo-hexahydro-1*H*-thieno[3,4-*d*]imidazol-4-yl)pentanamido) hexanamido)hexanamido)-3-phenylpropanamido)-2-phenylethylphosphonate (**Biotin-FFP**) was synthesized by the coupling of biotinamidohexanoyl-6-aminohexanoic acid N-hydroxysuccinimide ester (Biotin-X-X-NHS) with **H₂N-FFP.** The Biotin-X-X-NHS (10 µmol, 5.5 mg) was dissolved in dimethylsulfoxide (DMSO, 0.1 mL) and stirred on an ice bath. 1-Hydroxybenzotriazole (HOBt, 0.68 mg) added, followed by the addition of DIPEA (4 µL) after several minutes. The **H₂N-FFP** (5 mg, 8.1 µmol) was added as solution in DMSO (0.4 mL) and the reaction mixture left to warm at room temperature. The reaction was allowed to proceed overnight and monitored with TLC. When the reaction was completed the mixture was diluted with dichloromethane (15 mL) and wash with distilled water. The aqueous phase was washed with dichloromethane (15 mL) and the combined organic phases washed sequentially with aqueous citric acid (10%, 20 mL x 3), aqueous NaHCO₃ (5%, 20 mL x 3), H₂O (20 mL) and brine (20 mL), dried (Na₂SO₄), filtered, and concentrated *in vacuo,* to give, after purification by column chromatography (silica gel, Chloroform/Methanol 95:5 to 90:10), the desired biotinylated **Biotin-FFP** as pale white solid (4 mg, yield 52%). ¹H-NMR (600 MHz, 298 K, CD₃OD): *δ* 7.42-6.87 (m, 20H), 5.95-5.87 (m, 1H), 4.92-4.89 (m, 1H), 4.47-4.55 (m, 1H), 4.29-4.25 (m, 1H), 3.46-3.43 (m, 2H), 3.18-2.91 (m, 8H), 2.71-2.68 (m, 2H), 2.19-2.13 (m, 4H), 2.05 (t, 2H), 1.74-1.56 (m, 7), 1.53-1.28 (m, 14H). ³¹P-NMR (243 MHz, 298 K, CDCl₃): *δ* 17.20, 17.13. ESI-MS: *m*/*z* calcd for **Biotin-FFP** (C₅₁H₆₅N₆O₈PS) [M + Na]⁺ 975.41; found 975.36.

### EXAMPLE 3

### Specificity of inhibitor Boc-FFP and activity-based probe Biotin-FFP

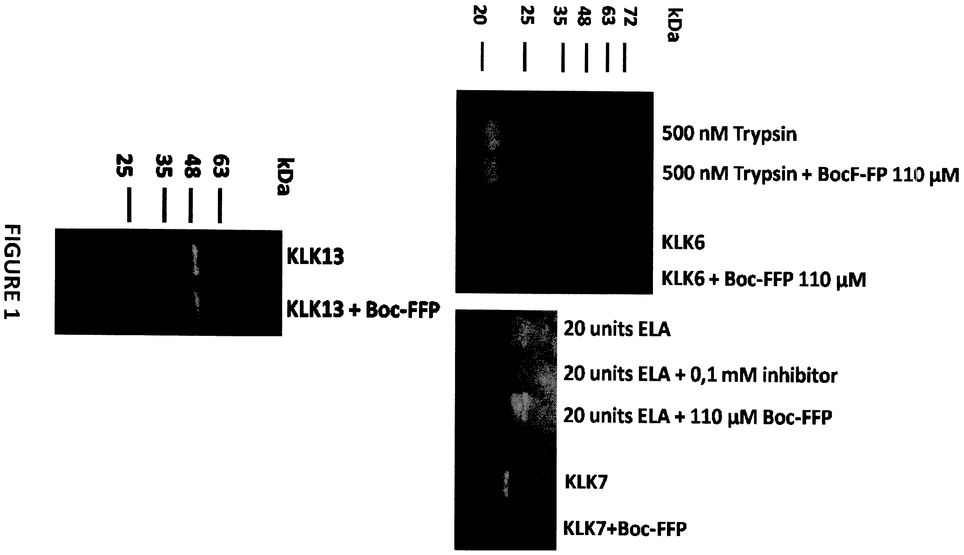

Specificity was tested with gel zymography against trypsin, elastase (ELA), KLK6 and KLK13. Only KLK7 was inhibited by Boc-FFP (**Figure 1**). Further in enzyme kinetic study Boc-FFP did not inhibit the activity of trypsin or KLK6 (**Table 1**).

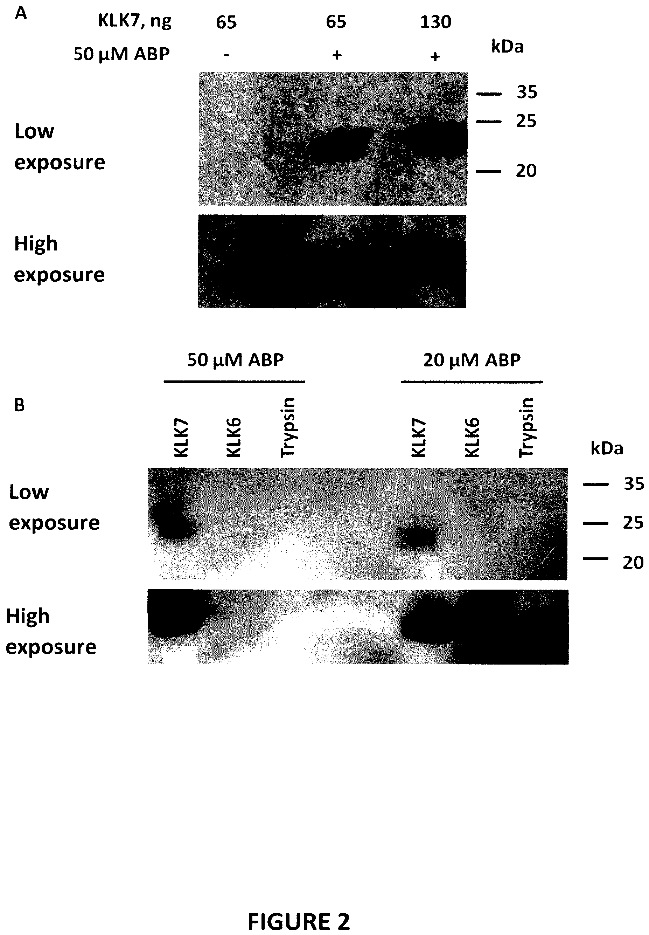

Similarly, the activity-based probe only detected the active KLK7 and not the trypsin or the KLK6 in a western-like blotting (**Figure 2**).

**Table 1 - Trypsin and KLK6 by Boc-FFP. Boc-FFP was pre-incubated with the enzymes for 2 hours. Aprotinin was used as positive control. Data were expressed as the change in fluorescence signal over a period of 3 min.**

| Sample | ΔF/Δt |
|---|---|
| Trypsin | 33.49 |
| Trypsin + Boc-FFP (2 h) | 33.26 |
| Trypsin + Aprotinin | 0 |
| KLK6 | 35.54 |
| KLK6 + Boc-FFP (2 h) | 37.96 |
| KLK6 + Aprotinin | 24.56 |

IC₅₀ has been determined as described below and found 1.91 µM.

For gel zymography, the compounds were reacted with the enzymes at room temperature for 2 hours, then mixed with zymogram loading buffer without mercaptoethanol, incubated at 37°C for 15 min and resolved on 12% SDS-PAGE containing 0.1% casein or gelatin substrates. Gels were washed twice with 50 mM Tris-HCI pH 7.5, 5 mM CaCl₂, 2.5% Triton X-100 for 15 min, then 15 min with 50 mM Tris-HCI, pH 7.5, 5 mM CaCl₂, 0.1% Triton X-100, and finally incubated in the latter buffer for 24 hours and stained with Coomassie G-250. Proteolytic bands appear white against a blue background.

The enzyme kinetic study testing the ability of Boc-FFP to inhibit trypsin or KLK6 was performed with cleavage of fluorescent substrate Z-Phe-Arg-AMC. The enzymes were used at 12 nM and the substrate at 50 µM. Boc-FFP was used at 1.2 µM (100:1 molar ration inhibitor: enzyme). Aprotinin was used a positive control at 500 nM final concentration. Fluorescence was measured (λ_{exc}=380 nm, λₑₘ=440 nm) for 3 min and the results were expressed as ΔF/Δt.

For Western-like blotting, the enzymes were reacted with Biotin-FFP for 2 or 16 hours, resolved on SDS-PAGE, then, the proteins were transferred on PVDF membranes. Membranes were blocked with 3% BSA in PBS for 40 min at room temperature, washed twice with PBS and incubated for 1 hour at room temperature with streptavidin-HRP polymer (1:2,000) and subsequently washed four times with PBS containing 0.05% Tween-20. Specific bands were identified with ECL.

For IC₅₀ determination, KLK7 inhibition study was carried out in assay buffer (100 mM Tris-HCI, pH 7.8, 100 mM NaCl, 10 mM CaCl₂, 0.005% Triton X-100). KLK7 (6 nM) was incubated with the inhibitor (50 nM to 10 µM) for 16 hours at room temperature in the assay buffer. Then, the BODIPY-FL casein substrate (Molecular Probes) was added at 30 µg/ml and fluorescence was measured (λ_{exc}=485 nm, λₑₘ=530 nm). After 3 hours the fluorescence was measured again. Data were inserted into the online available program Very Simple IC50 Tool (http://www.ic50.tk/) for fitting and calculation of the IC₅₀.

### EXAMPLE 4

### The length of carbon atoms at P1 position determines the inhibitory activity against KLK7

Three different compounds that all have the same core phosphonate structure but different lengths of carbon atom separating the phenyl side group from the α-carbon of the α-aminomethylphosphonate resembling the P1 position (as depicted below), were designed and tested. Specifically compounds Boc-FFP, Boc-FbFP and Boc-FcFP were generated and tested for their ability to inhibit KLK7 with *in situ* zymography (casein).

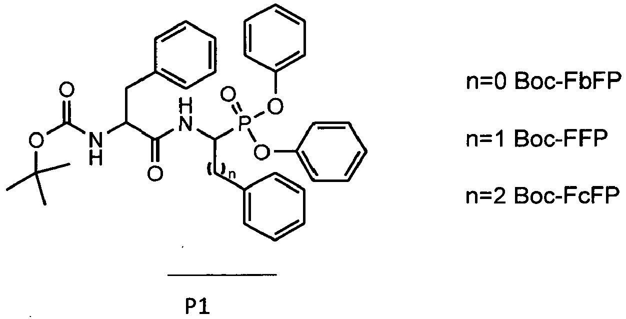

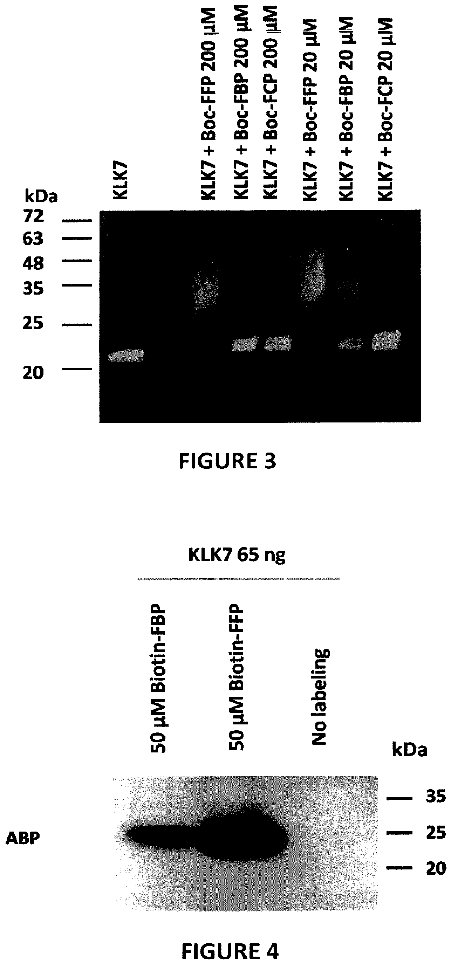

As shown in **Figure 3**, only Boc-FFP could effectively inhibit KLK7 activity, thus highlighting that small changes in the structure can substantially affect their ability to bind to KLK7. This was further confirmed after labeling KLK7 with Biotin-FFP and Biotin-FbFP in a Western-like assay. As shown in **Figure 4** and in accordance to findings in Figure 3, Biotin-FFP labeling of KLK7 results in higher signal compared to Biotin-FbFP.

### EXAMPLE 4

### Localization of the KLK7 activity in healthy skin biopsy and biopsies obtained from Netherton syndrome patients

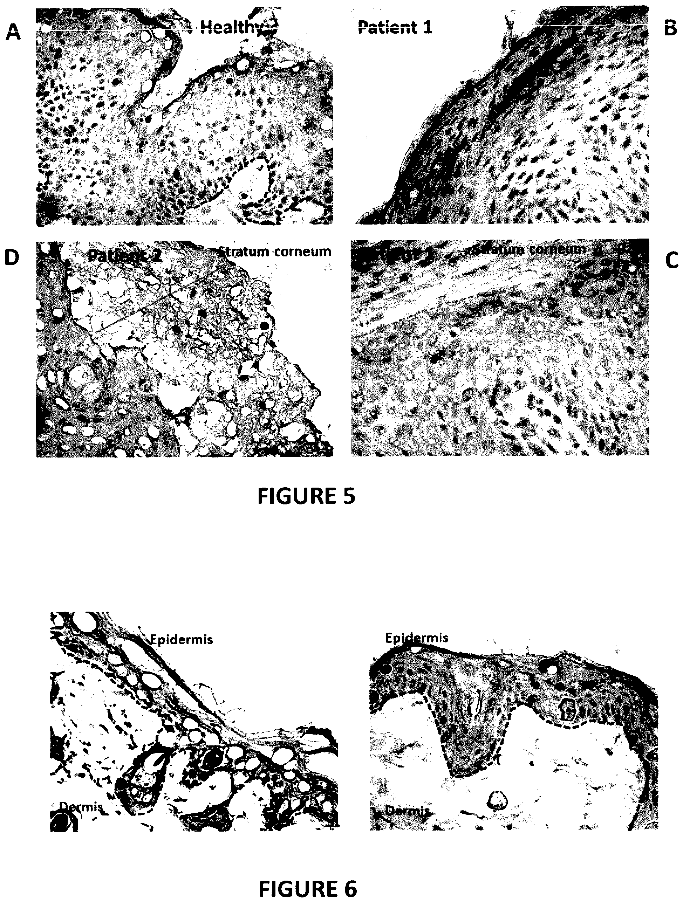

The activity-based probe Biotin-FFP was used to localize the KLK7 activity in healthy skin biopsy and in biopsies obtained from two Netherton syndrome patients (**Figure 5**) using activography. In normal healthy skin staining was observed at the superficial stratum corneum. In contrast, in NS samples staining was observed at the regions of stratum corneum rupture or all over the stratum corneum. Further staining was evident at the interface of stratum corneum/stratum granulosum.

The activity-based probe Biotin-FFP was also used to investigate the expression of KLK7 in mouse skin induced by 12-O-tetradecanoylphorbol 13-acetate (TPA), a classical chemical irritant and tumour promoter. Indeed, as shown in **Figure 6****,** TPA highly induces the expression of active KLK7.

Clinical specimens (skin biopsies were taken from a healthy volunteer and two patients with Netherton syndrome), embedded in OCT (optimal cutting temperature) and stored at -80°C until cryosectioned. All human subjects provided written consents.

For TPA induction, mice treated with 12-O-tetradecanoylphorbol 13-acetate were shaved and 24 hours later 100 µl of 0.0002 M TPA in acetone was applied on their right flank. Acetone alone was applied on the left flank as negative control. TPA application was repeated once more, 24 hours later and mice were sacrificed 24 hours after the second application, skin biopsy was taken, embedded in OCT (optimal cutting temperature) and stored at -80°C until sectioned with a cryotome.

### EXAMPLE 5

### Development of an ELISA to quantify the levels of active KLK7.

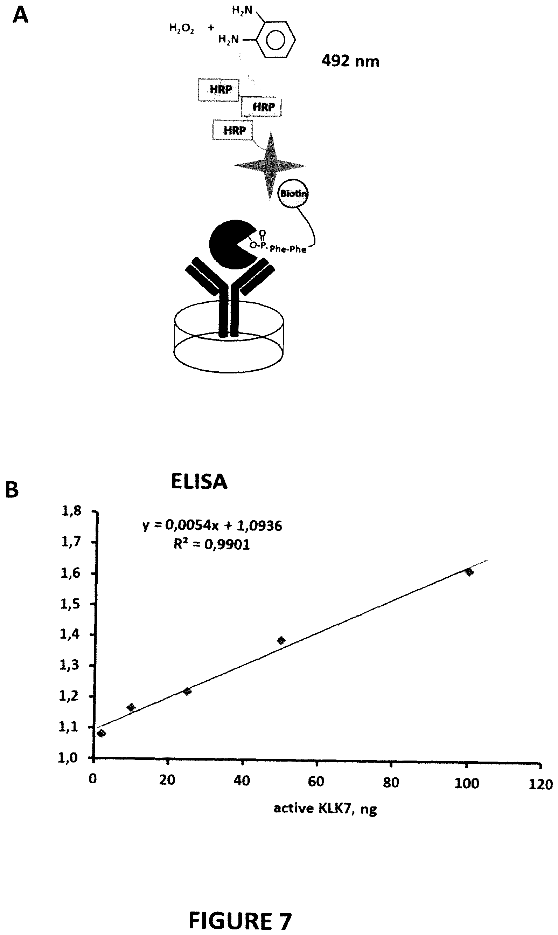

An Enzyme-Linked Immunosorbent Assay (ELISA) was developed to determine the levels of active KLK7 in biological or clinical specimens. The schematic diagram of the ELISA and the quantification are shown in **Figure 7****.**

Each well of a 96-well plate was incubated for 16 hours at 4°C with 500 ng of anti-KLK7 antibody in 100 µl of Phosphate Buffered Saline (PBS) as coating buffer (pH 7.4) Meanwhile KLK7 in several concentrations and Biotin-FFP (6.3 µM) were left to react for 16 hours in room temperature. The plate was washed two times with PBS (200 µl/well) and the blocking of remaining protein-binding sites in the coated wells was achieved by adding 200 µl/well blocking buffer (1% Bovine Serum Albumin (BSA) in PBS) for 1 hour at room temperature. Then the plate was washed four times with PBS (200 µl/well) followed by addition of the Biotin-FFP/KLK7 reaction mixture and left for incubation for 16 hours at room temperature. The plate was washed for 2 times with PBS and 100 µl of freshly prepared solution of streptavidin peroxidase polymer (1 ng/µl) were added in each well and incubated for 1 hour at room temperature followed by washing with PBS+0.005% Tween20 (200 µl/well, 4 times). The plate was washed two times with 200 µl/well of citrate buffer 100 mM (pH 6.0) and color development was achieved in the dark with 100 µl/well solution of 2 mg/ml OPD + 1µl/ml H₂O₂ 30% in citrate buffer 100 mM for 4 minutes. 100 µl/well stop solution (2M H₂SO₄) was added and the absorbance (optical density at 492 nm) was measured immediately in an Infinite F50 Tecan instrument.

### EXAMPLE 6

### Boc-FFP inhibits desquamation and inflammation in Spink5^{-/}⁻Klk5^{-/-} mice

Boc-FFP and Biotin-FFP did not exhibit either erythema or oedema *in vivo* when applied at concentration of 1 mM on the skin of wildtype (wt) or *Klk5*^{*-*/*-*} mice as shown in Table 4 and Table 5. Scoring was based on the following criteria (Table 2 and 3):

**Table 2 - Scoring criteria for erythema and eschar formation**

| **Erythema and Eschar Formation** | **Score** |
|---|---|
| No erythema | 0 |
| Very slight erythema (barely perceptible) | 1 |
| Well defined erythema | 2 |
| Moderate to severe erythema | 3 |
| Severe erythema (beef redness) | 4 |

**Table 3 - Scoring criteria for oedema formation**

| **Oedema Formation** | **Score** |
|---|---|
| No oedema | 0 |
| Very slight oedema (barely perceptible) | 1 |
| Slight oedema (edges of area well defined by definite raising) | 2 |
| Moderate oedema (raised approximately 1mm) | 3 |
| Severe erythema (raised more than 1mm and extending beyond area of exposure) | 4 |

**Table 4 - Erythema and eschar score formation after application of Boc-FFP or Biotin-FFP. Vehicle (solvent) treatment showed 0 score in all time points.**

| | | **Erythema and Eschar Score after (hrs)** | | | |
|---|---|---|---|---|---|
| **Genotype** | **Substance** | **4** | **24** | **48** | **72** |
| wt | Boc-FFP | 0 | 0 | 0 | 0 |
| | Biotin-FFP | 0 | 0 | 0 | 0 |
| *Klk5*^{*-*/*-*} | Boc-FFP | 0 | 0 | 0 | 0 |
| | Biotin-FFP | 0 | 0 | 0 | 0 |

**TABLE 5. Oedema score formation after application of Boc-FFP or Biotin-FFP. Vehicle (solvent) treatment showed 0 score in all time points.**

| | | **Oedema Score after (hrs)** | | | |
|---|---|---|---|---|---|
| **Genotype** | **Substance** | **4** | **24** | **48** | **72** |
| wt | Boc-FFP | 0 | 0 | 0 | 0 |
| | Biotin-FFP | 0 | 0 | 0 | 0 |
| *Klk5*^{*-*/*-*} | Boc-FFP | 0 | 0 | 0 | 0 |
| | Biotin-FFP | 0 | 0 | 0 | 0 |

Thus, Boc-FFP and Biotin-FFP lack toxicity in wild type and *Klk5*^{*-*/*-*}mice.

Application of Boc-FFP on the skin of *Spink5*^{*-*/}*⁻Klk5*^{*-*/*-*} mice resulted in reduction of macroscopic desquamation by approximately 50% (**Figure 8**). Further, it suppressed the expression of inflammatory cytokines (**Figure 9**).

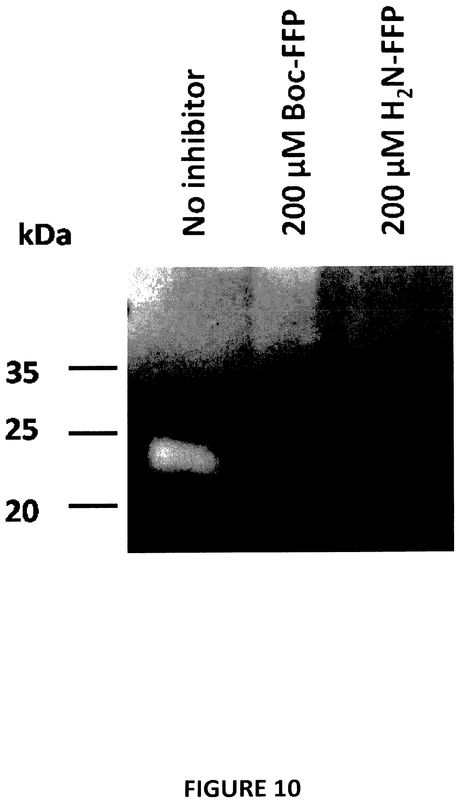

Finally, as shown in **Figure 10** the free H2N-FFP, like the protected Boc-FFP, can also inhibit the KLK7 activity.

## Claims

1. A compound of formula (I) having a KLK7 inhibitory activity 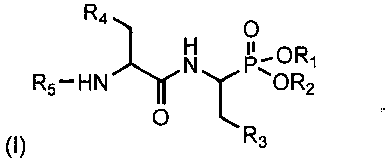 wherein
R1 and R2 are independently selected from branched or unbranched: C₁₋₆ alkyl; heteroalkyl; C₂₋₇ alkenyl; C₂₋₇ alkynyl; C₆₋₁₁ cycloalkyl; heterocyclyl; C₆₋₁₁ aryl; C₆₋₁₁ aryl-C₁₋₆ alkyl; heteroaryl; heteroaryl-C₁₋₆ alkyl; C₁₋₆ alkoxy; heterocyclyloxy; C₂₋₇ acyl; C₂₋₇ acyloxy; C₁₋₆ alkoxycarbonyl; naphthyl; or anthracenyl group; wherein each may be unsubstituted or substituted by one or more of halogen, hydroxy, amino, aminoalkyl, thiol, cyano, nitro, oxo, carboxy, C₁₋₃ alkyl, carboxyalkyl or C₁₋₃ alkoxy group; or R1 is C₁₋₂₀ alkyl or C₃₋₂₀ cycloalkyl group, preferably methyl, ethyl, propyl or isopropyl and R2 is a C₆₋₁₁ aryl that may be unsubstituted or substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher;
R3 and R4 are independently selected from phenyl, para-hydroxyphenyl, para-fluorophenyl, para-trifluoromethyl, pentafluorophenyl, 2,4-difluorophenyl, 2,3-difluorophenyl, 3,4-difluorophenyl, 2,5-difluorophenyl, 2,6-difluorophenyl, 2,3,4-trifluorophenyl, 2,3,5-trifluorophenyl, 2,3,6-trifluorophenyl, 3,4,5-trifluorophenyl, 2,4,6-trifluorophenyl, 2,3,4,5-tetrafluorophenyl, 2,3,4,6-tetrafluorophenyl, or 2,3,5,6-tetrafluorophenyl group;
R5 is independently selected from a hydrogen atom, Boc, Fmoc or Z-group, or a label, wherein the label is selected from the group consisting of a biotin that is linked to the amino group via a spacer comprising -NH-C₁₋₂₀ alkyl-C(=0)-, NH-C₁₋₂₀ alkyl-C(=O)-NH-C₁₋₂₀ alkyl-C(=O)-, -NH-CH₂-CH₂-(O-CH₂-CH₂)ₙ-C(=O)- wherein n=1 to 6, or a random co-polymer of -NH-C₁₋₂₀ alkyl-C(=0)- and -NH-CH₂-CH₂-(O-CH₂-CH₂)ₙ-C(=O)-connected via amide bonds, wherein C₁₋₂₀ alkyl is branched or unbranched; a fluorescing moiety such as fluorescein, Cy5, Cy5.5, Sulfo-Cy5 or BODIPY; digoxygenin; a radioactive moiety; or an immunohistochemically detectable moiety; or R5 is a click-reactive functional group selected from the group comprising an alkyne, cyclooctyne derivative, azide derivative, alkene, hydrazine, hydrazine derivative, alkoxyamine, alkoxyamine derivative, aminoxy and thiol group;
with the proviso that, when R5 is a fluorescing moiety, then R2 is a C₆₋₁₁ aryl that may be unsubstituted or substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher, and R1 is a C₁₋₂₀ alkyl or C₃₋₂₀ cycloalkyl group, preferably methyl, ethyl, propyl or isopropyl;
with the proviso that, when R2 is a C₆₋₁₁ aryl that may be unsubstituted or substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher, then R5 is a fluorescing moiety and R1 is a C₁₋₂₀ alkyl or C₃₋₂₀ cycloalkyl group, preferably methyl, ethyl, propyl or isopropyl;
or pharmaceutically acceptable salt, isomer, enantiomer, tautomer or racemic mixture thereof;
for use in the treatment or prevention of a skin disorder **characterized by** abnormal desquamation and/or inflammation, an inflammatory disease, a neurodegenerative disease or cancer.

2. The compound of formula (I) for use according to claim 1, wherein R1 and R2 are independently selected from branched or unbranched: C₁₋₆alkyl, C₅₋₁₁ cycloalkyl, phenyl, phenyl-C₁₋₆ alkyl, C₁₋₆ alkoxy, C₁₋₆ acyl, C₂₋₆ acyloxy or C₁₋₆ alkoxycarbonyl group, wherein each may be unsubstituted or substituted by one or more of halogen, hydroxy, amino, nitro, oxo, carboxy, C₁₋₃ alkyl, carboxyalkyl or C₁₋₃ alkoxy group; or R1 is a C₁₋₂₀ alkyl or C₃₋₂₀ cycloalkyl group, preferably methyl, ethyl, propyl or isopropyl and R2 is a C₆₋₁₁ aryl that may or may not be substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher.

3. The compound of formula (I) for use according to claim 1, wherein R1 and R2 are independently selected from phenyl, nitrophenyl, chlorophenyl, dinitrophenyl, dichlorophenyl, difluoroethyl or trifluoroethyl group; or R1 is a C₁₋₂₀ alkyl or C₃₋₂₀ cycloalkyl group, preferably methyl, ethyl, propyl or isopropyl and R2 is a phenyl, chlorophenyl or dichlorophenyl group, each further substituted with an amino, aminoalkyl(C₁₋₂₀), carboxy or carboxyalkyl(C₁₋₂₀) group covalently attached to a fluorescent quencher.

4. The compound of formula (I) for use according to claim 1, wherein said compound is tert-butyl 1-(1-(diphenoxyphosphoryl)-2-phenylethylamino)-1-oxo-3-phenylpropan-2-ylcarbamate, diphenyl 1-(2-(6-(6-(5-((4R)-2-oxo-hexahydro-1*H-*thieno[3,4-*d*]imidazole-4-yl)pentanamido)hexanamido)hexanamido)-3-phenylpropanamido)-2-phenylethylphosphonate or diphenyl 1-(2-amino-3-phenylpropanamido)-2-phenyl ethylphosphonate.

5. The compound of formula (I) according to anyone of claims 1 to 4, for use in the treatment of a skin disorder selected from the group comprising Netherton syndrome or other ichthyotic skin disorders such as autosomal dominant ichthyosis, X-linked ichthyosis or epidermolytic hyperkeratosis, atopic dermatitis, psoriasis and acne rosacea.

6. The compound of formula (I) according to anyone of claims 1 to 4, for use in the treatment of neurodegenerative disease selected from the group comprising amyotrophic lateral sclerosis, Parkinson's disease, Alzheimer's, Huntington's disease and ataxia telangiectasia.

7. The compound of formula (I) according to anyone of claims 1 to 4, for use in the treatment of cancer, wherein said cancer is selected from the group comprising ovarian cancer, pancreatic cancer, melanoma, cervical cancer, papillary thyroid cancer, gliomas, oral squamous cell carcinoma, prostate cancer, renal cell carcinoma and colon cancer.

8. The compound of formula (I) for use according to any one of claims 1 to 7, in combination with a therapeutic agent that inhibits the activity of KLK5.

9. The compound of formula (I) for use according to anyone of claims 1 to 7, wherein R5 is a label selected from the group comprising a biotin that is linked to the amino group via a spacer comprising -NH-C₁₋₂₀ alkyl-C(=0)-, NH-C₁₋₂₀ alkyl-C(=O)-NH-C₁₋₂₀ alkyl-C(=O)-, -NH-CH₂-CH₂-(O-CH₂-CH₂)ₙ-C(=O)- wherein n=1 to 6, or a random co-polymer of -NH-C₁₋₂₀ alkyl-C(=O)- and -NH-CH₂-CH₂-(O-CH₂-CH₂)ₙ-C(=O)-connected via amide bonds, wherein C₁₋₂₀ alkyl is branched or unbranched; a fluorescing moiety such as fluorescein, Cy5, Cy5.5, Sulfo-Cy5 or BODIPY; digoxygenin; a radioactive moiety; or an immunohistochemically detectable moiety, or R5 is a click-reactive functional group selected from the group comprising an alkyne, cyclooctyne derivative, azide derivative, alkene, hydrazine, hydrazine derivative, alkoxyamine, alkoxyamine derivative, aminoxy and thiol group;

10. The compound of formula (I) for use according to claim 9, wherein said use further comprises a method of obtaining an in vivo image of said compound in the cell or tissue that is being treated.

11. The compound of formula (I) for use according to claim 10, wherein
R1 is a C₁₋₂₀ alkyl or C₃₋₂₀ cycloalkyl group, preferably methyl, ethyl, propyl or isopropyl;
R2 is a C₆₋₁₁ aryl that may or may not be substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher; preferably a phenyl, chlorophenyl or dichlorophenyl group, each further substituted with an amino, aminoalkyl(C₁₋₂₀), carboxy or carboxyalkyl(C₁₋₂₀) group covalently attached to a fluorescent quencher; and
R5 is a fluorescing moiety such as fluorescein, Cy5, Cy5.5, Sulfo-Cy5 or BODIPY.

12. The compound of formula (I) for use according to claim 10 or 11, wherein said method of obtaining an in vivo image of said compound in the cell or tissue being treated comprises (a) allowing the compound to distribute within the subject after administration, (b) detecting the label, and (c) correlating the presence, the levels, distribution and/or localization of said label to disease status; and wherein,
when R5 is biotin that is linked to the amino group via a spacer comprising -NH-C₁₋₂₀ alkyl-C(=0)-, NH-C₁₋₂₀ alkyl-C(=O)-NH-C₁₋₂₀ alkyl-C(=O)-, -NH-CH₂-CH₂-(O-CH₂-CH₂)ₙ-C(=O)- wherein n=1 to 6, or a random co-polymer of -NH-C₁₋₂₀alkyl-C(=0)- and -NH-CH₂-CH₂-(O-CH₂-CH₂)ₙ-C(=O)- connected via amide bonds, wherein C₁₋₂₀ alkyl is branched or unbranched, the method further comprises, after step (a), administering to the subject a second compound comprising streptavidin carrying a fluorescing moiety;
when R5 is a click-reactive functional group, the method further comprises, after step (a), administering to the subject a second compound comprising a click-reactive functional group that is complementary to the R5 click-reactive functional group and wherein the second compound further comprises a detectable label selected from the group comprising biotin, a fluorescing moiety, digoxygenin, a paramagnetic substance or magnetic particle, an electron dense reagent, a radioactive moiety and an immunohistochemically detectable moiety.

13. Cosmetic composition comprising at least one of the compounds as defined in any one of claims 1 to 4 or 9 and a cosmetically acceptable carrier.

14. The cosmetic composition according to claim 13, wherein the concentration of said compound(s) is between 0.001% and 10% by weight relative to the whole composition.

15. Use of a cosmetic composition as defined in claims 13 or 14 for body or hair care, preferably for the improvement of an undesirable skin condition.

16. A method for the classification, diagnosis or prognosis of a skin disorder **characterized by** abnormal desquamation and/or inflammation, an inflammatory disease, a neurodegenerative disease or cancer, or for monitoring response to a medical treatment, in a sample such as a cell extract, cell, tissue sample, bodily fluid, tissue homogenate or other, comprising (a) contacting said sample with the compound of formula (I), wherein
R1 and R2 are as defined in anyone of claims 1 to 3;
R3 and R4 are independently selected from phenyl, para-hydroxyphenyl, para-fluorophenyl, para-trifluoromethyl, pentafluorophenyl, 2,4-difluorophenyl, 2,3-difluorophenyl, 3,4-difluorophenyl, 2,5-difluorophenyl, 2,6-difluorophenyl, 2,3,4-trifluorophenyl, 2,3,5-trifluorophenyl, 2,3,6-trifluorophenyl, 3,4,5-trifluorophenyl, 2,4,6-trifluorophenyl, 2,3,4,5-tetrafluorophenyl, 2,3,4,6-tetrafluorophenyl, or 2,3,5,6-tetrafluorophenyl group;
R5 is a label selected from the group comprising a biotin that is linked to the amino group via a spacer comprising -NH-C₁₋₂₀ alkyl-C(=0)-, NH-C₁₋₂₀ alkyl-C(=O)-NH-C₁₋₂₀alkyl-C(=O)-, -NH-CH₂-CH₂-(O-CH₂-CH₂)ₙ-C(=O)- wherein n=1 to 6, or a random co-polymer of -NH-C₁₋₂₀ alkyl-C(=0)- and -NH-CH₂-CH₂-(O-CH₂-CH₂)ₙ-C(=O)- connected via amide bonds, wherein C₁₋₂₀ alkyl is branched or unbranched; a fluorescing moiety such as fluorescein, Cy5, Cy5.5, Sulfo-Cy5 or BODIPY; digoxygenin; a radioactive moiety; or an immunohistochemically detectable moiety;
with the proviso that, when R2 is a C₆₋₁₁ aryl that may or may not be substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher; preferably a phenyl, chlorophenyl or dichlorophenyl group, each further substituted with an amino, aminoalkyl(C₁₋₂₀), carboxy or carboxyalkyl(C₁₋₂₀) group covalently attached to a fluorescent quencher, then R5 is a fluorescing moiety and R1 is a C₁₋₂₀ alkyl or C₃₋₂₀ cycloalkyl group, preferably methyl, ethyl, propyl or isopropyl;
or salt, isomer, enantiomer, tautomer or racemic mixture thereof;
(b) determining the presence, the levels and/or localization of said label, (c) comparing the presence, the levels and/or localization of said label to a reference sample or to a sample from the same subject prior to treatment, and (d) correlating the differences in the presence, the levels, distribution and/or localization of said label to disease status or response to treatment.

17. A compound of formula (I) for use in a method of in vivo imaging of a skin disorder **characterized by** abnormal desquamation and/or inflammation, an inflammatory disease, a neurodegenerative disease or cancer, wherein
R1 and R2 are as defined in anyone of claims 1 to 3;
R3 and R4 are independently selected from phenyl, para-hydroxyphenyl, para-fluorophenyl, para-trifluoromethyl, pentafluorophenyl, 2,4-difluorophenyl, 2,3-difluorophenyl, 3,4-difluorophenyl, 2,5-difluorophenyl, 2,6-difluorophenyl, 2,3,4-trifluorophenyl, 2,3,5-trifluorophenyl, 2,3,6-trifluorophenyl, 3,4,5-trifluorophenyl, 2,4,6-trifluorophenyl, 2,3,4,5-tetrafluorophenyl, 2,3,4,6-tetrafluorophenyl, or 2,3,5,6-tetrafluorophenyl group;
R5 is a detectable label selected from the group comprising a biotin that is linked to the amino group via a spacer comprising -NH-C₁₋₂₀ alkyl-C(=0)-, NH-C₁₋₂₀ alkyl-C(=O)-NH-C₁₋₂₀ alkyl-C(=O)-, -NH-CH₂-CH₂-(O-CH₂-CH₂)ₙ-C(=O)- wherein n=1 to 6, or a random co-polymer of -NH-C₁₋₂₀ alkyl-C(=0)- and -NH-CH₂-CH₂-(O-CH₂-CH₂)ₙ-C(=O)-connected via amide bonds, wherein C₁₋₂₀ alkyl is branched or unbranched; a fluorescing moiety such as fluorescein, Cy5, Cy5.5, Sulfo-Cy5 or BODIPY; digoxygenin; a radioactive moiety; or an immunohistochemically detectable moiety, or R5 is a click-reactive functional group selected from the group comprising an alkyne, cyclooctyne derivative, azide derivative, alkene, hydrazine, hydrazine derivative, alkoxyamine, alkoxyamine derivative, aminoxy and thiol group;
with the proviso that, when R5 is a fluorescing moiety then R2 is a C₆₋₁₁ aryl that may or may not be substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher; preferably a phenyl, chlorophenyl or dichlorophenyl group, each further substituted with an amino, aminoalkyl(C₁₋₂₀), carboxy or carboxyalkyl(C₁₋₂₀) group covalently attached to a fluorescent quencher, and R1 is a C₁₋₂₀ alkyl or C₃₋₂₀ cycloalkyl group, preferably methyl, ethyl, propyl or isopropyl;
with the proviso that, when R2 is a C₆₋₁₁ aryl that may or may not be substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher; preferably a phenyl, chlorophenyl or dichlorophenyl group, each further substituted with an amino, aminoalkyl(C₁₋₂₀), carboxy or carboxyalkyl(C₁₋₂₀) group covalently attached to a fluorescent quencher, then R5 is a fluorescing moiety and R1 is a C₁₋₂₀ alkyl or C₃₋₂₀ cycloalkyl group, preferably methyl, ethyl, propyl or isopropyl;
or pharmaceutically acceptable salt, isomer, enantiomer, tautomer or racemic mixture thereof.

18. The compound of formula (I) for use according to claim 17, wherein
R1 is a C₁₋₂₀ alkyl or C₃₋₂₀ cycloalkyl group, preferably methyl, ethyl, propyl or isopropyl;
R2 is a C₆₋₁₁ aryl that may or may not be substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher; preferably a phenyl, chlorophenyl or dichlorophenyl group, each further substituted with an amino, aminoalkyl(C₁₋₂₀), carboxy or carboxyalkyl(C₁₋₂₀) group covalently attached to a fluorescent quencher; and
R5 is a fluorescing moiety such as fluorescein, Cy5, Cy5.5, Sulfo-Cy5 or BODIPY.

19. The compound of formula (I) for use according to claim 17, wherein
R1 and R2 are independently selected from branched or unbranched: C₁₋₆alkyl, C₅₋₁₁ cycloalkyl, phenyl, phenyl-C₁₋₆ alkyl, C₁₋₆alkoxy, C₁₋₆ acyl, C₂₋₆ acyloxy or C₁₋₆ alkoxycarbonyl group, wherein each may be unsubstituted or substituted by one or more of halogen, hydroxy, amino, nitro, oxo, carboxy, C₁₋₃ alkyl, carboxyalkyl or C₁₋₃ alkoxy group;
R5 is a click-reactive functional group selected from the group comprising an alkyne, cyclooctyne derivative, azide derivative, alkene, hydrazine, hydrazine derivative, alkoxyamine, alkoxyamine derivative, aminoxy and thiol group.

20. The compound of formula (I) for use according to anyone of claims 17 to 19, wherein the method comprises: (a) administering the compound to a subject, (b) allowing the compound to distribute within the subject, and (c) detecting the label; and wherein, when R5 is a click-reactive functional group, the method further comprises, after step (b), administering to the subject a second compound comprising a click-reactive functional group that is complementary to the R5 click-reactive functional group and wherein the second compound further comprises a detectable label selected from the group comprising a radioactive label, a fluorophore, a paramagnetic substance or magnetic particle, an electron dense reagent, biotin and digoxigenin.

21. A kit for use in the treatment, prevention or diagnosis of a skin disorder **characterized by** abnormal desquamation and/or inflammation, an inflammatory disease, a neurodegenerative disease and cancer, comprising (a) the compound of formula (I) having a KLK7 inhibitory activity, wherein
R1 and R2 are independently selected from branched or unbranched: C₁₋₆alkyl, C₅₋₁₁ cycloalkyl, phenyl, phenyl-C₁₋₆ alkyl, C₁₋₆ alkoxy, C₁₋₆ acyl, C₂₋₆ acyloxy or C₁₋₆ alkoxycarbonyl group, wherein each may be unsubstituted or substituted by one or more of halogen, hydroxy, amino, nitro, oxo, carboxy, C₁₋₃ alkyl, carboxyalkyl or C₁₋₃ alkoxy group;
R3 and R4 are independently selected from phenyl, para-hydroxyphenyl, para-fluorophenyl, para-trifluoromethyl, pentafluorophenyl, 2,4-difluorophenyl, 2,3-difluorophenyl, 3,4-difluorophenyl, 2,5-difluorophenyl, 2,6-difluorophenyl, 2,3,4-trifluorophenyl, 2,3,5-trifluorophenyl, 2,3,6-trifluorophenyl, 3,4,5-trifluorophenyl, 2,4,6-trifluorophenyl, 2,3,4,5-tetrafluorophenyl, 2,3,4,6-tetrafluorophenyl, or 2,3,5,6-tetrafluorophenyl group;
R5 is a click-reactive functional group selected from the group comprising an alkyne, cyclooctyne derivative, azide derivative, alkene, hydrazine, hydrazine derivative, alkoxyamine, alkoxyamine derivative, aminoxy and thiol group;
or pharmaceutically acceptable salt, isomer, enantiomer, tautomer or racemic mixture thereof, in a suitable carrier;
(b) a second compound comprising a click-reactive functional group that is complementary to the R5 click-reactive functional group and wherein said second compound further comprises a detectable label selected from the group comprising biotin, a fluorescing moiety, digoxygenin, a paramagnetic substance or magnetic particle, an electron dense reagent, a radioactive moiety, or an immunohistochemically detectable moiety, in a suitable carrier;
and optionally reagents and/or instructions for use.

22. A compound of formula (I) having a KLK7 inhibitory activity 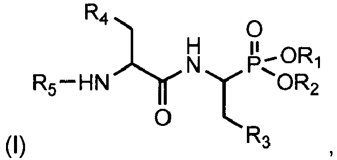 wherein
R1 and R2 are independently selected from branched or unbranched: C₁₋₆alkyl; heteroalkyl; C₂₋₇ alkenyl; C₂₋₇ alkynyl; C₆₋₁₁ cycloalkyl; heterocyclyl; C₆₋₁₁ aryl; C₆₋₁₁ aryl-C₁₋₆ alkyl; heteroaryl; heteroaryl-C₁₋₆ alkyl; C₁₋₆ alkoxy; heterocyclyloxy; C₂₋₇ acyl; C₂₋₇ acyloxy; C₁₋₆ alkoxycarbonyl; naphthyl; or anthracenyl group; wherein each may be unsubstituted or substituted by one or more of halogen, hydroxy, amino, aminoalkyl, thiol, cyano, nitro, oxo, carboxy, C₁₋₃ alkyl, carboxyalkyl or C₁₋₃ alkoxy group; or R1 is a C₁₋₂₀ alkyl or C₃₋₂₀ cycloalkyl group, preferably methyl, ethyl, propyl or isopropyl and R2 is a C₆₋₁₁ aryl that may be unsubstituted or substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher;
R3 and R4 are independently selected from phenyl, para-hydroxyphenyl, para-fluorophenyl, para-trifluoromethyl, pentafluorophenyl, 2,4-difluorophenyl, 2,3-difluorophenyl, 3,4-difluorophenyl, 2,5-difluorophenyl, 2,6-difluorophenyl, 2,3,4-trifluorophenyl, 2,3,5-trifluorophenyl, 2,3,6-trifluorophenyl, 3,4,5-trifluorophenyl, 2,4,6-trifluorophenyl, 2,3,4,5-tetrafluorophenyl, 2,3,4,6-tetrafluorophenyl, or 2,3,5,6-tetrafluorophenyl group;
R5 is a label selected from the group consisting of a biotin that is linked to the amino group via a spacer comprising -NH-C₁₋₂₀ alkyl-C(=0)-, NH-C₁₋₂₀ alkyl-C(=O)-NH-C₁₋₂₀ alkyl-C(=O)-, -NH-CH₂-CH₂-(O-CH₂-CH₂)ₙ-C(=O)- wherein n=1 to 6, or a random co-polymer of -NH-C₁₋₂₀ alkyl-C(=0)- and -NH-CH₂-CH₂-(O-CH₂-CH₂)ₙ-C(=O)- connected via amide bonds, wherein C₁₋₂₀ alkyl is branched or unbranched; a fluorescing moiety such as fluorescein, Cy5, Cy5.5, Sulfo-Cy5 or BODIPY; digoxygenin; a radioactive moiety; or an immunohistochemically detectable moiety; or R5 is a click-reactive functional group selected from the group comprising an alkyne, cyclooctyne derivative, azide derivative, alkene, hydrazine, hydrazine derivative, alkoxyamine, alkoxyamine derivative, aminoxy and thiol group;
with the proviso that, when R5 is a fluorescing moiety, then R2 is a C₆₋₁₁ aryl that may be unsubstituted or substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher, and R1 is a C₁₋₂₀ alkyl or C₃₋₂₀ cycloalkyl group, preferably methyl, ethyl, propyl or isopropyl;
with the proviso that, when R2 is a C₆₋₁₁ aryl that may be unsubstituted or substituted with a halogen group, wherein said C₆₋₁₁ aryl is further substituted with an amino, aminoalkyl(C₁₋₂₀), carboxy or carboxyalkyl(C₁₋₂₀) group covalently attached to a fluorescent quencher, then R5 is a fluorescing moiety and R1 is a C₁₋₂₀ alkyl or C₃₋₂₀ cycloalkyl group, preferably methyl, ethyl, propyl or isopropyl;
or pharmaceutically acceptable salt, isomer, enantiomer, tautomer or racemic mixture thereof.
